# EUROPEAN PATENT APPLICATION

(11) **EP 2 357 177 A1**
(43) Date of publication of application: **17.08.2011**
(21) Application number: 09822015.5
(22) Date of filing: 20.10.2009
(51) Int. Cl.: C07D 221/18, C07C 213/00, C07C 215/68, C07C 227/22, C07C 229/52, C07C 271/30

(54) **HELICENE DERIVATIVE, AXIALLY ASYMMETRIC AMINO ACID, AMINE OR AMINOALCOHOL DERIVATIVE, PERYLENE DERIVATIVE OR SALT THEREOF, AND METHODS FOR PRODUCING SAME**

(30) Priority: 24.10.2008 JP 2008273761; 11.09.2009 JP 2009210278
(71) Applicant: Kyoto University, Sakyo-ku Kyoto-shi Kyoto 606-8501 (JP)
(72) Inventor: FURUTA, Takumi, Uji-shi Kyoto 611-0011 (JP); KAWABATA, Takeo, Uji-shi Kyoto 611-0011 (JP)
(74) Representative: Wiedemann, Peter
(86) International application number: PCT/JP2009/068043
(87) International publication number: WO 2010/047318

(57) **Abstract**

An object of the present invention is to provide a novel helicene derivative, axially chiral amino acid, amine or amino alcohol derivative, and azaperylene.

The present inventors developed compounds represented by Formulae (I), (I'), (II), (II'), and (III) : 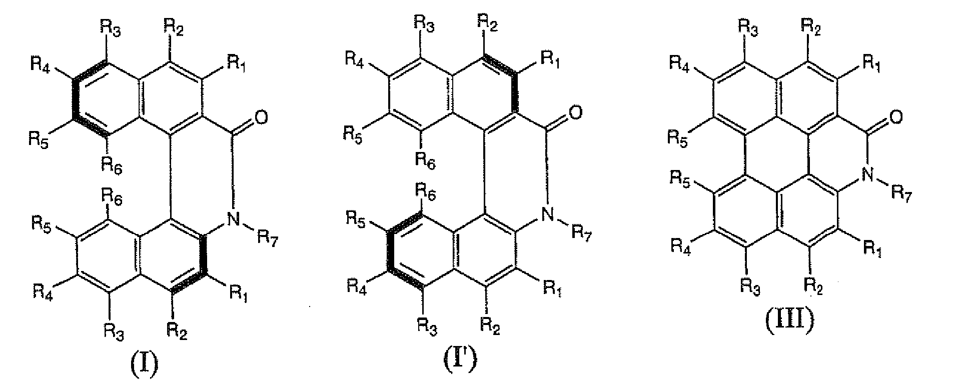 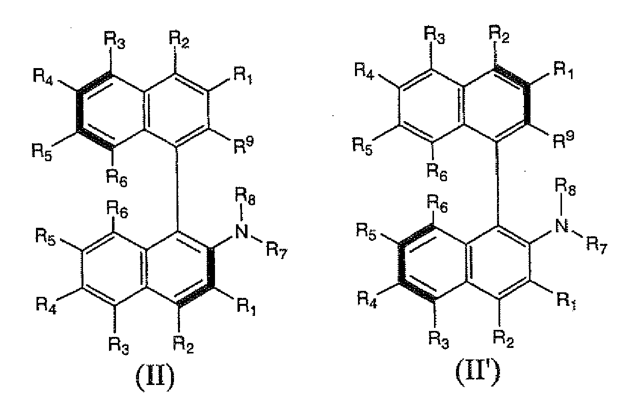 and a method for producing the same.

## Description

### Technical Field

The present invention relates to a helicene derivative, an axially chiral amino acid, amine or amino alcohol derivative, a perylene derivative, or salts thereof, and a method for producing the same.

### Background Art

Helicenes are useful molecules as asymmetric catalysts, asymmetric molecule recognition elements, and organic materials having specific optical properties, and there has been a demand for the development of novel derivatives thereof. However, the synthesis thereof is not always easy. A photocyclization reaction has been widely used as a method for synthesizing a helicene. For example, a stilbene derivative synthesized by a Wittig reaction is photocyclized. It is difficult to synthesize helicenes in large quantities, and they are often synthesized through a complicated process. Accordingly, there has been a demand for the development of a method for synthesizing a helicene derivative without using a photocyclization reaction (see Patent Literature 1).

Among axially chiral compounds, axially chiral amino acids, amines, or amino alcohols having an amino group and a carboxy group or alcohol group in the molecules are expected to have wide applications as asymmetric organic catalysts, chiral ligands, and chiral building blocks. However, there are not many examples of their syntheses (see Patent Literature 2), and there has been a demand for the creation of a novel axially chiral amino acid, amine or amino alcohol, and the development of an efficient synthesis method thereof.

Also in regard to perylene derivatives useful as organic optical materials (such as organic EL elements), fluorescent reagents, and the like, there has been a demand for the creation of a novel derivative, and the development of an efficient synthesis method thereof.

### Citation List

### Patent Literatures

[PTL 1] Japanese Unexamined Patent Publication No. 2008-69104
[PTL 2] Japanese Unexamined Patent Publication No. 2006-143627

### Summary of Invention

### Technical Problem

The present inventors found a novel helicene derivative, axially chiral amino acid, amine, or amino alcohol derivative, and perylene derivative, as well as a method for efficiently synthesizing them; and completed the present invention.

Specifically, the present invention provides a novel helicene derivative, axially chiral amino acid, amine, or amino alcohol derivative, and perylene derivative; as well as a method for producing the same, as described below.

### Item 1.

A helicene derivative or salt thereof represented by Formula (I) or (I'):

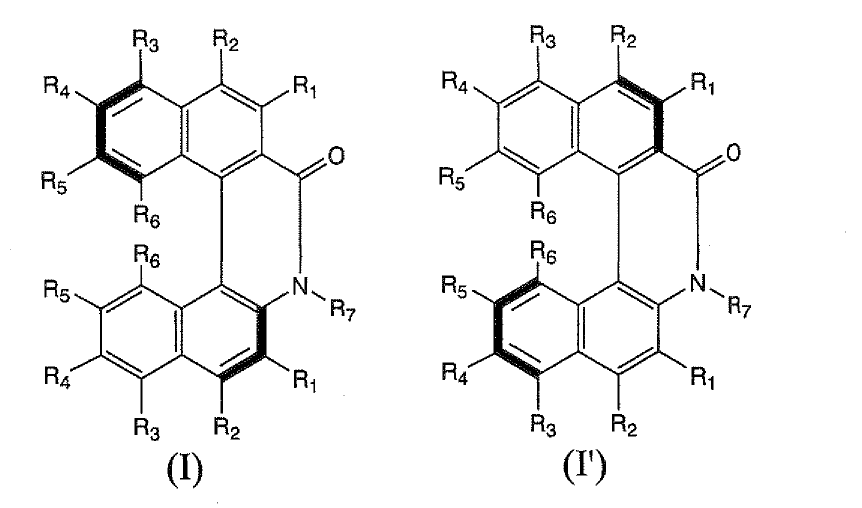

wherein R₁, R₂, R₃, R₄, R₅, and R₆ may be the same or different, and represent hydrogen, alkyl, cycloalkyl, aryl, heteroaryl, alkenyl, alkynyl, cyano, alkoxy, hydroxy, acyloxy, CONH₂, monoalkylaminocarbonyl, dialkylaminocarbonyl, nitro, amino, monoalkylamino, dialkylamino, alkanoylamino, azido, carboxy (COOH), alkoxycarbonyl, or acyl; two adjacent groups among R₁, R₂, R₃, R₄, R₅, and R₆ may be joined together with the carbon atoms to which they are attached to form a 5- or 6-membered saturated or unsaturated ring structure or structures, and these ring structures may be substituted; and R₇ represents hydrogen, alkyl, cycloalkyl, aryl, heteroaryl, acyl, or alkoxycarbonyl.

### Item 2.

An axially chiral amino acid, amine, or amino alcohol derivative, or salt thereof, represented by Formula (II) or (II'):

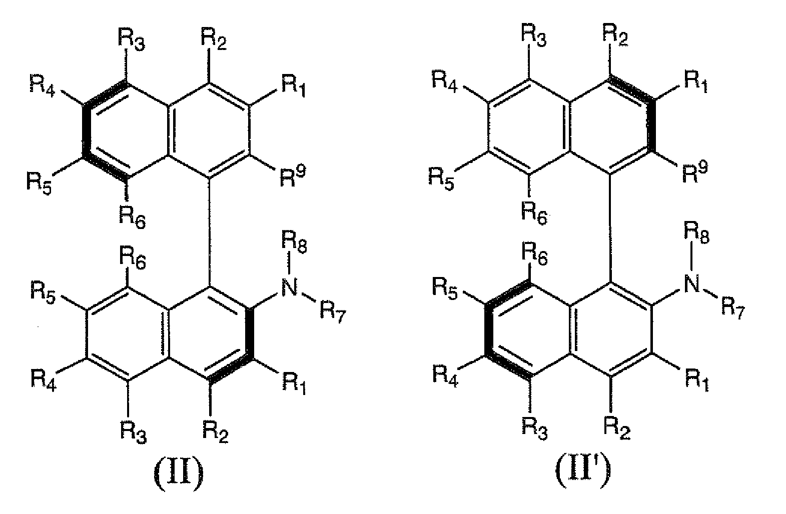

wherein R₁, R₂, R₃, R₄, R₅, and R₆ may be the same or different, and represent hydrogen, alkyl, cycloalkyl, aryl, heteroaryl, alkenyl, alkynyl, cyano, alkoxy, hydroxy, acyloxy, CONH₂, monoalkylaminocarbonyl, dialkylaminocarbonyl, nitro, amino, monoalkylamino, dialkylamino, alkanoylamino, azido, carboxy (COOH), alkoxycarbonyl, or acyl; two adjacent groups among R₁, R₂, R₃, R₄, R₅, and R₆ may be joined together with the carbon atoms to which they are attached to form a 5- or 6-membered saturated or unsaturated ring structure or structures, and these ring structures may be substituted; R₇ and R₈ each independently represent hydrogen, alkyl, aryl, heteroaryl, acyl, or alkoxycarbonyl; and R₉ represents COOZ, CONZ₂ or CZ₂OH (each Z is the same or different, and represents hydrogen, alkyl, cycloalkyl, aryl, heteroaryl, or aralkyl), CN, or tetrazole.

### Item 3.

A perylene derivative or salt thereof represented by Formula (III):

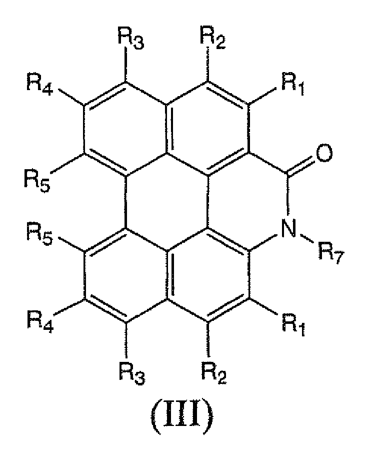

wherein R₁, R₂, R₃, R₄, and R₅ may be the same or different, and represent hydrogen, alkyl, cycloalkyl, aryl, heteroaryl, alkenyl, alkynyl, cyano, alkoxy, hydroxy, acyloxy, CONH₂, monoalkylaminocarbonyl, dialkylaminocarbonyl, nitro, amino, monoalkylamino, dialkylamino, alkanoylamino, azido, carboxy (COOH), alkoxycarbonyl, or acyl; two adjacent groups among R₁, R₂, R₃, R₄, and R₅ may be joined together with the carbon atoms to which they are attached to form a 5- or 6-membered saturated or unsaturated ring structure or structures, and these ring structures may be substituted; and R₇ represents hydrogen, alkyl, cycloalkyl, aryl, heteroaryl, acyl, or alkoxycarbonyl.

### Item 4.

A method for producing a helicene derivative represented by Formula (I) or (I'):

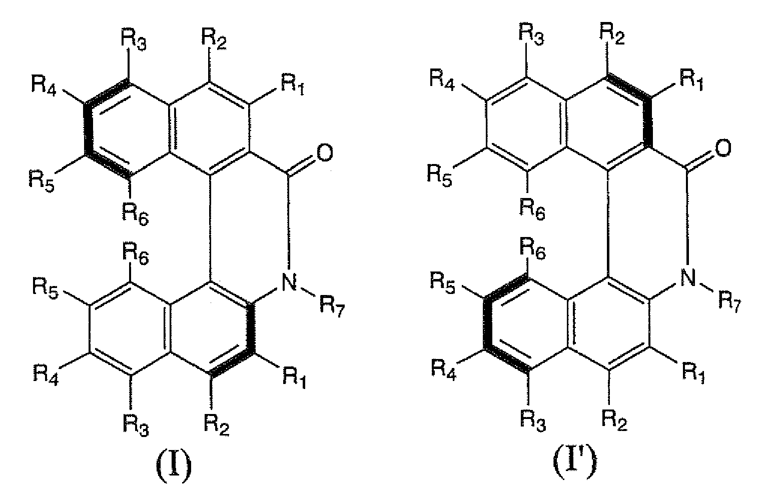

wherein R₁ to R₇ are as defined below,
the method comprising reacting, with a Pd catalyst, a compound represented by Formula (IV):

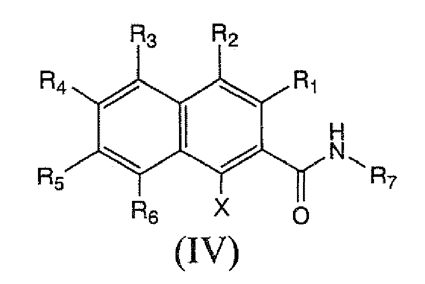

wherein
R₁, R₂, R₃, R₄, R₅, and R₆ may be the same or different, and represent hydrogen, alkyl, cycloalkyl, aryl, heteroaryl, alkenyl, alkynyl, cyano, alkoxy, hydroxy, acyloxy, CONH₂, monoalkylaminocarbonyl, dialkylaminocarbonyl, nitro, amino, monoalkylamino, dialkylamino, alkanoylamino, azido, carboxy (COOH), alkoxycarbonyl, or acyl; two adjacent groups among R₁, R₂, R₃, R₄, R₅, and R₆ may be joined together with the carbon atoms to which they are attached to form a 5- or 6-membered saturated or unsaturated ring structure or structures, and these ring structures may be substituted; R₇ represents hydrogen, alkyl, cycloalkyl, aryl, heteroaryl, acyl, or alkoxycarbonyl; and X represents iodine, bromine, chlorine, or triflate.

### Item 5.

A method for producing an axially chiral amino acid, amine, or amino alcohol derivative represented by Formula (II) or (II'):

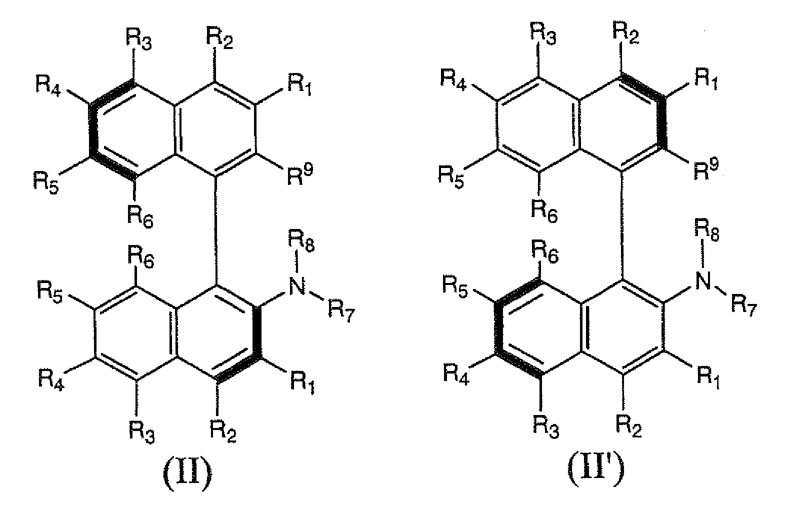

wherein
R₁, R₂, R₃, R₄, R₅, and R₆ may be the same or different, and represent hydrogen, alkyl, cycloalkyl, aryl, heteroaryl, alkenyl, alkynyl, cyano, alkoxy, hydroxy, acyloxy, CONH₂, monoalkylaminocarbonyl, dialkylaminocarbonyl, nitro, amino, monoalkylamino, dialkylamino, alkanoylamino, azido, carboxy (COOH), alkoxycarbonyl, or acyl; two adjacent groups among R₁, R₂, R₃, R₄, R₅, and R₆ may be joined together with the carbon atoms to which they are attached to form a 5- or 6-membered saturated or unsaturated ring structure or structures, and these ring structures may be substituted; R₇ and R₈ each independently represent hydrogen, alkyl, aryl, heteroaryl, acyl, or alkoxycarbonyl; and R₉ represents COOZ, CONZ₂ or CZ₂OH (each Z is the same or different, and represents hydrogen, alkyl, cycloalkyl, aryl, heteroaryl, or aralkyl), CN, or tetrazole,
the method comprising opening a lactam ring in the compound represented by Formula (I) or (I') in Claim 1, using a metal hydroxide; converting, if necessary, the resulting carboxy (COOH) to COOZ, CONZ₂ or CZ₂OH (each Z is the same or different, and represents hydrogen, alkyl, cycloalkyl, aryl, heteroaryl, or aralkyl), CN, or tetrazole; and converting, if necessary, an amino group (NH₂) to NR₇R₈, wherein R₇ and R₈ each independently represent hydrogen, alkyl, aryl, heteroaryl, acyl, or alkoxycarbonyl.

### Item 6.

A method for converting, using a sulfonic acid, a compound represented by Formula (Ia) or (Ia'):

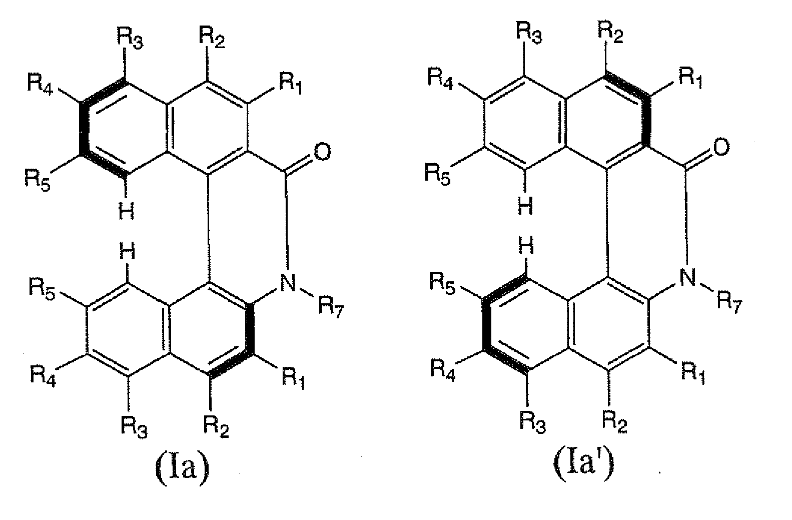

wherein R₁, R₂, R₃, R₄, and R₅ may be the same or different, and represent hydrogen, alkyl, cycloalkyl, aryl, heteroaryl, alkenyl, alkynyl, cyano, alkoxy, hydroxy, acyloxy, CONH₂, monoalkylaminocarbonyl, dialkylaminocarbonyl, nitro, amino, monoalkylamino, dialkylamino, alkanoylamino, azido, carboxy (COOH), alkoxycarbonyl, or acyl; two adjacent groups among R₁, R₂, R₃, R₄, and R₅ may be joined together with the carbon atoms to which they are attached to form a 5- or 6-membered saturated or unsaturated ring structure or structures, and these ring structures may be substituted; and R₇ represents hydrogen, alkyl, cycloalkyl, aryl, heteroaryl, acyl, or alkoxycarbonyl,
to a perylene derivative represented by Formula (III):

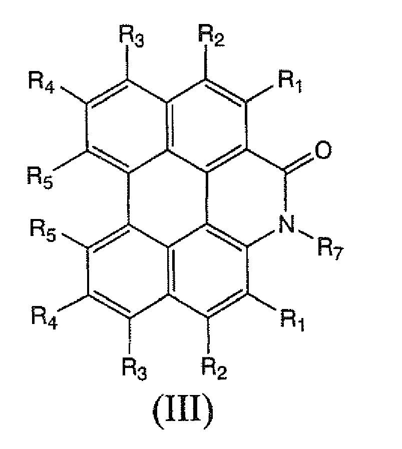

wherein R₁ to R₅ and R₇ are as defined above.

In each formula above, R₁, R₂, R₃, R₄, R₅, and R₆ may be the same or different; and examples thereof include hydrogen, alkyl, cycloalkyl, aryl, heteroaryl, alkenyl, alkynyl, cyano, alkoxy, hydroxy, acyloxy, CONH₂, monoalkylaminocarbonyl, dialkylaminocarbonyl, nitro, amino, monoalkylamino, dialkylamino, alkanoylamino, azido, carboxy (COOH), alkoxycarbonyl, and acyl. Preferable examples of R₁, R₂, R₃, R₄, R₅, and R₆ include hydrogen, methyl, phenyl, alkenyl, alkynyl, cyano, methylether, hydroxy, nitro, amino, azido, carboxy, methoxycarbonyl, acetyl, chlorine, bromine, and iodine. Further, any two adjacent groups among R₁, R₂, R₃, R₄, R₅, and R₆ may be joined together to form a 5- or 6-membered saturated or unsaturated ring structure or structures, and these ring structures may be substituted. The two adjacent groups described herein refer to any of the following combinations: R₁ and R₂, R₂ and R₃, R₃ and R₄, R₄ and R₅, and R₅ and R₆.

Further, in each formula above, R₇ represents a substituent such as hydrogen, alkyl, cycloalkyl, aryl, heteroaryl, acyl, alkoxycarbonyl, or the like. Examples thereof include methyl, ethyl, n-propyl, n-butyl, benzyl, p-methoxybenzyl, phenyl, p-methoxyphenyl, acetyl, t-butyloxycarbonyl (Boc), benzyloxycarbonyl (Cbz), allyloxycarbonyl (Alloc), etc.

### Advantageous Effects of Invention

According to the present invention, a novel helicene derivative, axially chiral amino acid, amine, or amino alcohol derivative, and perylene derivative can be efficiently synthesized.

### Description of Embodiments

The present invention provides the helicene derivative or salt thereof represented by Formula (I) or (I'):

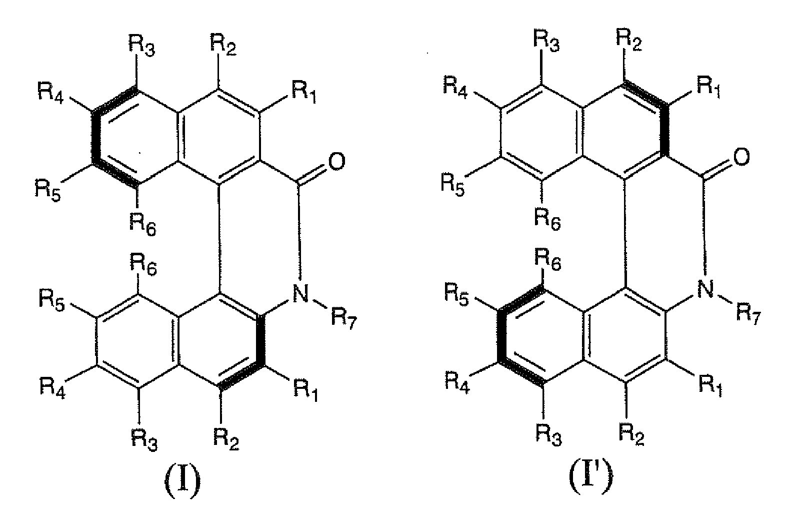

wherein R₁, R₂, R₃, R₄, R₅, and R₆ may be the same or different, and represent hydrogen, alkyl, cycloalkyl, aryl, heteroaryl, alkenyl, alkynyl, cyano, alkoxy, hydroxy, acyloxy, CONH₂, monoalkylaminocarbonyl, dialkylaminocarbonyl, nitro, amino, monoalkylamino, dialkylamino, alkanoylamino, azido, carboxy (COOH), alkoxycarbonyl, or acyl; two adjacent groups among R₁, R₂, R₃, R₄, R₅, and R₆ may be joined together with the carbon atoms to which they are attached to form a 5- or 6-membered saturated or unsaturated ring structure or structures, and these ring structures may be substituted; and R₇ represents hydrogen, alkyl, cycloalkyl, aryl, heteroaryl, acyl, or alkoxycarbonyl.

The salts of the compounds presented by Formulae (I) to (IV) mean acid addition salts of compounds having a group (amino groups such as amino, monoalkylamino, and dialkylamino) capable of forming an acid addition salt in the structure), or base salts of the compounds of Formulae (I) to (IV) having a group (COOH, SO₃H, phenolic OH, etc.) capable of forming a salt with a base in the structure. Specific examples of acid addition salts include inorganic acid salts such as hydrochloride, hydrobromide, hydroiodide, sulfate, perchlorate, and phosphate; organic acid salts such as oxalate, malonate, succinate, maleate, fumarate, lactate, malate, citrate, tartrate, benzoate, trifluoroacetate, acetate, methanesulfonate, p-toluenesulfonate and trifluoromethanesulfonate; and acidic amino acid salts such as glutamate and aspartate. Specific examples of base salts include alkali metal or alkaline earth metal salts such as sodium salt, potassium salt, or calcium salt; salts with organic bases such as pyridine salt and triethylamine salt; and salts with basic amino acids such as lysine and arginine.

Example of the "5- or 6-membered saturated or unsaturated ring structure or structures" include hydrocarbon ring structures such as cyclopentane, cyclohexane, cyclopentene, cyclohexene, and benzene; and ring structures containing a hetero atom such as pyrrole, pyrrolidine, piperidine, piperazine, pyridine, pyrimidine, pyridazine, imidazole, furan, pyran, dihydrofuran, dihydropyran, tetrahydropyran, tetrahydrofuran, thiophene, thiazole, and oxazol. Hydrocarbon ring structures are preferred. Examples of substituents for the ring structure include halogens (F, Br, Cl, I), alkyl, aryl, alkoxy, amino, cyano, etc. The number of substituents is 3 or less, preferably 2 or less, more preferably 1 or 0.

The "alkyl group" may be linear, branched, or cyclic. Examples thereof include C₁₋₁₀ alkyl groups such as methyl, ethyl, n-propyl, isopropyl, n-butyl, isobutyl, tert-butyl, n-pentyl, isopentyl, hexyl, heptyl, octyl, nonyl, and decyl; preferably C₁₋₆ alkyl groups; more preferably C₁₋₄ alkyl groups.

Examples of the "cycloalkyl group" include C₃₋₇ cycloalkyl groups such as cyclopropyl, cyclobutyl, cyclopentyl, cyclohexyl, and cycloheptyl.

The "heteroaryl group" means a monocyclic or polycyclic group comprising a 5- or 6-membered aromatic ring or rings, which contain 1 to 3 hetero atoms selected from N, O, and S. When polycyclic, at least one ring is aromatic. Specific examples thereof include furyl, thienyl, pyrrolyl, imidazolyl, pyrazolyl, oxazolyl, thiazolyl, isoxazolyl, isothiazolyl, pyridyl, pyrazinyl, pyrimidinyl, pyridazinyl, indolyl, quinolyl, isoquinolyl, benzo[b]thienyl, and benzimidazolyl.

Examples of the "aralkyl group" include benzyl, phenethyl, propylphenyl, diphenylmethyl, and naphthylmethyl.

The "alkenyl group" may be linear, branched, or cyclic, and has at least one double bond. Examples thereof include C₂₋₁₀ alkenyl groups such as vinyl, allyl, 1-propenyl, 2-methyl-2-propenyl, isopropenyl, 1-, 2- or 3-butenyl, 2-, 3- or 4-pentenyl, 2-methyl-2-butenyl, 3-methyl-2-butenyl, 5-hexenyl, 1-cyclopentenyl, 1-cyclohexenyl, 3-methyl-3-butenyl; preferably C₂₋₆ alkenyl groups; more preferably C₂₋₄ alkenyl groups.

The "alkynyl group" may be linear, branched, or cyclic, and has at least one triple bond. Examples thereof include C₂₋₁₀ alkynyl groups such as ethynyl, 1- or 2-propynyl, 1-, 2- or 3-butynyl, and 1-methyl-2-propynyl; preferably C₂₋₆ alkynyl groups; more preferably C₂₋₄ alkynyl groups.

Examples of the "monoalkylamino, group" include amino groups mono-substituted with C₁₋₆ alkyl groups, such as methylamino, ethylamino, n-propylamino, isopropylamino, n-butylamino, isobutylamino, tert-butylamino, n-pentylamino, isopentylamino, and hexylamino.

Examples of the "dialkylamino group" include amino groups di-substituted with C₁₋₆ alkyl groups, such as dimethylamino, diethylamino, di-n-propylamino, diisopropylamino, di-n-butylamino, diisobutylamino, di-tert-butylamino, di-n-pentylamino, diisopentylamino, and dihexylamino.

Examples of the "monoalkylaminocarbonyl group" include aminocarbonyl groups mono-substituted with C₁₋₆ alkyl groups, such as methylaminocarbonyl, ethylaminocarbonyl, n-propylaminocarbonyl, isopropylaminocarbonyl, n-butylaminocarbonyl, isobutylaminocarbonyl, tert-butylaminocarbonyl, n-pentylaminocarbonyl, isopentylaminocarbonyl, and hexylaminocarbonyl.

Examples of the "dialkylaminocarbonyl group" include aminocarbonyl groups di-substituted with C₁₋₆ alkyl, such as dimethylaminocarbonyl, diethylaminocarbonyl, di-n-propylaminocarbonyl, diisopropylaminocarbonyl, di-n-butylaminocarbonyl, diisobutylaminocarbonyl, di-tert-butylaminocarbonyl, di-n-pentylaminocarbonyl, diisopentylaminocarbonyl, and dihexylaminocarbonyl.

The "acyl group" means C₁₋₆ alkylcarbonyl, arylcarbonyl, or aryl-substituted C₁₋₄ alkylcarbonyl.

Examples of C₁₋₆ alkylcarbonyl include methylcarbonyl, ethylcarbonyl, n-propylcarbonyl, isopropylcarbonyl, n-butylcarbonyl, isobutylcarbonyl, tert-butylcarbonyl, n-pentylcarbonyl, isopentylcarbonyl, and hexylcarbonyl.

Examples of arylcarbonyl include phenylcarbonyl, naphthylcarbonyl, fluorenylcarbonyl, anthrylcarbonyl, biphenylylcarbonyl, tetrahydronaphthylcarbonyl, chromanylcarbonyl, 2,3-dihydro-1,4-dioxanaphthalenylcarbonyl, indanylcarbonyl, and phenanthrylcarbonyl.

Examples of aryl-substituted C₁₋₄ alkylcarbonyl include benzylcarbonyl, naphthylmethylcarbonyl, fluorenylmethylcarbonyl, anthrylmethylcarbonyl, biphenylylmethylcarbonyl, tetrahydronaphthylmethylcarbonyl, chromanylmethylcarbonyl, 2,3-dihydro-1,4-dioxanaphthalenylmethylcarbonyl, indanylmethylcarbonyl and phenanthrylmethylcarbonyl, phenethylcarbonyl, naphthylethylcarbonyl, fluorenylethylcarbonyl, anthrylethylcarbonyl, biphenylylethylcarbonyl, tetrahydronaphthylethylcarbonyl, chromanylethylcarbonyl, 2,3-dihydro-1,4-dioxanaphthalenylethylcarbonyl, indanylethylcarbonyl, and phenanthrylethylcarbonyl.

The "acyloxy group" means C₁₋₆ alkylcarbonyloxy, arylcarbonyloxy, or aryl-substituted C₁₋₄ alkylcarbonyloxy.

Examples of C₁₋₆ alkylcarbonyloxy include methylcarbonyloxy, ethylcarbonyloxy, n-propylcarbonyloxy, isopropylcarbonyloxy, n-butylcarbonyloxy, isobutylcarbonyloxy, tert-butylcarbanyloxy, n-pentylcarbonyloxy, isopentylcarbonyloxy, and hexylcarbonyloxy.

Examples of arylcarbonyloxy include phenylcarbonyloxy, naphthylcarbonyloxy, fluorenylcarbonyloxy, anthrylcarbonyloxy, biphenylylcarbonyloxy, tetrahydronaphthylcarbonyloxy, chromanylcarbonyloxy, 2,3-dihydro-1,4-dioxanaphthalenylcarbonyloxy, indanylcarbonyloxy, and phenanthrylcarbonyloxy.

Examples of aryl-substituted C₁₋₄ alkylcarbonyloxy include benzylcarbonyloxy, naphthylmethylcarbonyloxy, fluorenylmethylcarbonyloxy, anthrylmethylcarbonyloxy, biphenylylmethylcarbonyloxy, tetrahydronaphthylmethylcarbonyloxy, chromanylmethylcarbonyloxy, 2,3-dihydro-1,4-dioxanaphthalenylmethylcarbonyloxy, indanylmethylcarbonyloxy and phenanthrylmethylcarbonyloxy, phenethylcarbonyloxy, naphthylethylcarbonyloxy, fluorenylethylcarbonyloxy, anthrylethylcarbonyloxy, biphenylylethylcarbonyloxy, tetrahydronaphthylethylcarbonyloxy, chromanylethylcarbonyloxy, 2,3-dihydro-1,4-diaxanaphthalenylethylcarbonyloxy, indanylethylcarbonyloxy, and phenanthrylethylcarbonyloxy.

Examples of the "alkanoylamino group" include methylcarbonylamino, ethylcarbonylamino, n-propylcarbonylamino, isopropylcarbonylamino, n-butylcarbonylamino, isobutylcarbonylamino, tert-butylcarbonylamino, n-pentylcarbonylamino, isopentylcarbonylamino, and hexylcarbonylamino.

The "aryl group" means a monocyclic or polycyclic group comprising a 5- or 6-membered aromatic hydrocarbon ring or rings. Specific examples include phenyl, tolyl, xylyl, naphthyl, fluorenyl, anthryl, biphenylyl, tetrahydronaphthyl, chromanyl, 2,3-dihydro-1,4-dioxanaphthalenyl, indanyl, and phenanthryl.

Examples of the "alkoxy group" include C₁₋₆ alkoxy groups such as methoxy, ethoxy, propoxy, isopropoxy, butoxy, isobutoxy, tert-butoxy, pentyloxy, isopentyloxy, and hexyloxy.

Examples of the "alkoxycarbonyl group" include C₁₋₆ alkoxycarbonyl groups such as methoxycarbonyl, ethoxycarbonyl, propoxycarbonyl, isopropoxycarbonyl, butoxycarbonyl, isobutoxycarbonyl, tert-butoxycarbonyl, pentyloxycarbonyl, isopentyloxycarbonyl and hexyloxycarbonyl, allyloxycarbonyl, and benzyloxycarbonyl.

The present invention is characterized in that Formula (IV):

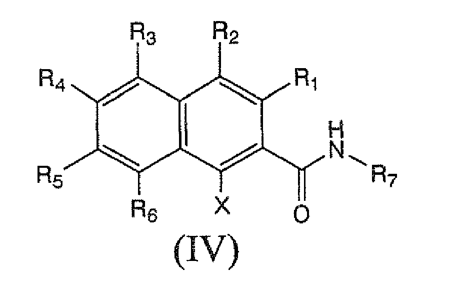

wherein R₁, R₂, R₃, R₄, R₅, and R₆ may be the same or different, and represent hydrogen, alkyl, cycloalkyl, aryl, heteroaryl, alkenyl, alkynyl, cyano, alkoxy, hydroxy, acyloxy, CONH₂, monoalkylaminocarbonyl, dialkylaminocarbonyl, nitro, amino, monoalkylamino, dialkylamino, alkanoylamino, azido, carboxy (COOH), alkoxycarbonyl, or acyl; two adjacent groups among R₁, R₂, R₃, R₄, R₅, and R₆ may be joined together with the carbon atoms to which they are attached to form a 5- or 6-membered saturated or unsaturated ring structure or structures, and these ring structures may be substituted; R₇ represents hydrogen, alkyl, cycloalkyl, aryl, heteroaryl, acyl, or alkoxycarbonyl; and X represents iodine, bromine, chlorine, or triflate, undergoes a homocoupling reaction with a Pd catalyst without adding ligands such as phosphine, thereby giving the helicene derivative shown by Formula (I) or (I'). The findings of the present invention are astonishing in view of the fact that a homocoupling reaction with a Pd catalyst usually requires a ligand.

The present invention provides the axially chiral amino acid, amine, or amino alcohol derivative represented by Formula (II) or (II'):

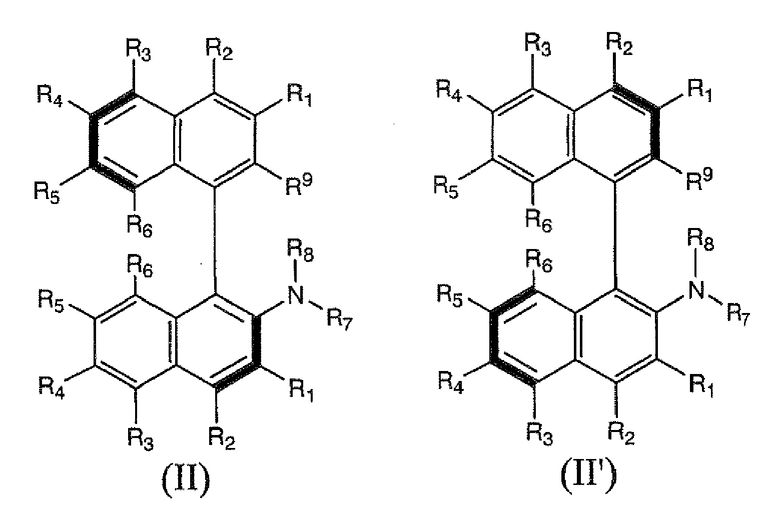

wherein R₁, R₂, R₃, R₄, R₅, and R₆ may be the same or different, and represent hydrogen, alkyl, cycloalkyl, aryl, heteroaryl, alkenyl, alkynyl, cyano, alkoxy, hydroxy, acyloxy, CONH₂, monoalkylazninocarbonyl, dialkylaminocarbonyl, nitro, amino, monoalkylamino, dialkylamino, alkanoylamino, azido, carboxy (COOH), alkoxycarbonyl, or acyl; two adjacent groups among R₁, R₂, R₃, R₄, R₅, and R₆ may be joined together with the carbon atoms to which they are attached to form a 5- or 6-membered saturated or unsaturated ring structure or structures, and these ring structures may be substituted; R₇ and R₈ each independently represent hydrogen, alkyl, aryl, heteroaryl, acyl, or alkoxycarbonyl; and R₉ represents COOZ, CONZ₂ or CZ₂OH (each Z is the same or different, and represents hydrogen, alkyl, cycloalkyl, aryl, heteroaryl, or aralkyl), CN, or tetrazole. The present invention is characterized in that, in the helicene derivative represented by Formula (I) or (I'):

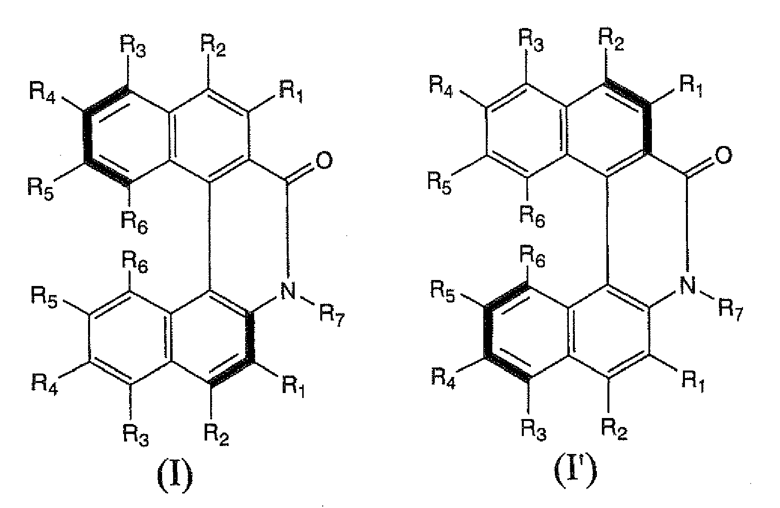

wherein R₁, R₂, R₃, R₄, R₅, and R₆ may be the same or different, and represent hydrogen, alkyl, cycloalkyl, aryl, heteroaryl, alkenyl, alkynyl, cyano, alkoxy, hydroxy, acyloxy, CONH₂, monoalkylaminocarbonyl, dialkylaminocarbonyl, nitro, amino, monoalkylamino, dialkylamino, alkanoylamino, azido, carboxy (COOH), alkoxycarbonyl, or acyl; two adjacent groups among R₁, R₂, R₃, R₄, R₅, and R₆ may be joined together with the carbon atoms to which they are attached to form a 5- or 6-membered saturated or unsaturated ring structure or structures, and these ring structures may be substituted; and R₇ represents hydrogen, alkyl, cycloalkyl, aryl, heteroaryl, acyl, or alkoxycarbonyl,
a lactam ring is opened by cleaving an amide bond in DMSO using an alkali metal hydroxide such as LiOH, NaOH, KOH, or the like, and if necessary, the resulting COOH group can be
(i) reduced using an appropriate reducing agent such as LiAlH₄, NaBH₄ or the like to obtain CH₂OH;
(ii) reacted with ZMgBr (Z represents alkyl, cycloalkyl, aryl, heteroaryl, or aralkyl) by a Grignard reaction to obtain CZ₂OH;
(iii) reacted with organic lithium Z-Li (Z represents alkyl, cycloalkyl, aryl, heteroaryl, or aralkyl) to obtain CZ₂OH;
(iv) converted as follows: COOH→CONH₂→CN→tetrazole (see Scheme 1 shown below); and
(v) reacted with ZOH or NHZ₂ in the presence of a coupling agent, dehydrating agent, or the like to obtain ester (COOZ) or amide (CONZ₂) ,
   or, NHR₇ produced by ring-opening is reacted with R₈-Y (Y represents a leaving group) in the presence of a base (NaH, t-butoxy potassium, etc.) to obtain NR₇R₈, thereby giving the axially chiral amino acid, amine, or amino alcohol derivative of Formula (II) or (II').

### <Scheme 1>

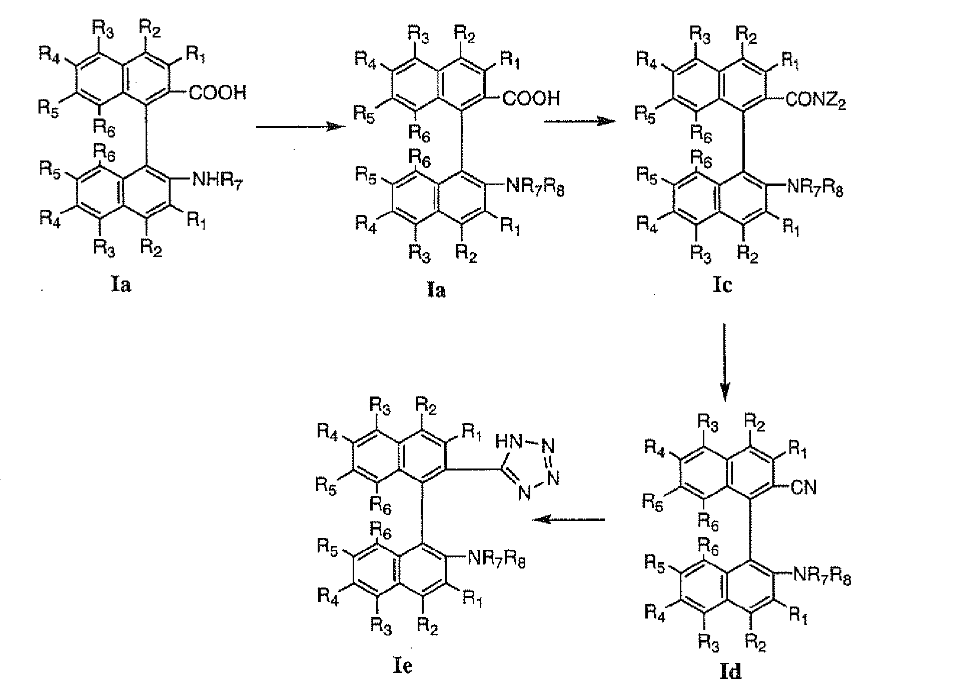

wherein R₁ to R₈ and Z are as defined above; and when Z is hydrogen, IC can be converted to Id by using a dehydrating agent).
The present invention provides the perylene derivative represented by Formula (III):

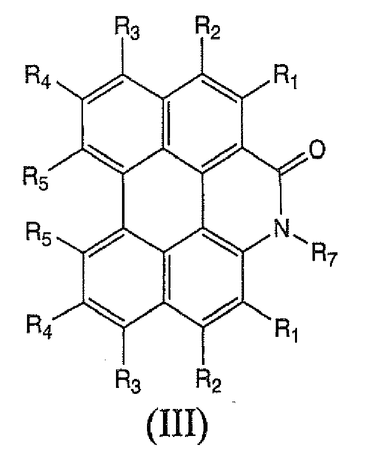

wherein R₁, R₂, R₃, R₄, and R₅ may be the same or different, and represent hydrogen, alkyl, cycloalkyl, aryl, heteroaryl, alkenyl, alkynyl, cyano, alkoxy, hydroxy, acyloxy, CONH₂, monoalkylaminocarbonyl, dialkylaminocarbonyl, nitro, amino, monoalkylamino, dialkylamino, alkanoylamino, azido, carboxy (COOH), alkoxycarbonyl, or acyl; two adjacent groups among R₁, R₂, R₃, R₄, and R₅ may be joined together with the carbon atoms to which they are attached to form a 5- or 6-membered saturated or unsaturated ring structure or structures, and these ring structures may be substituted; and R₇ represents hydrogen, alkyl, cycloalkyl, aryl, heteroaryl, acyl, or alkoxycarbonyl. The present invention is characterized in giving the perylene derivative of Formula (III) by intramolecular carbon-carbon bond formation by a sulfonic acid in the helicene derivative represented by Formula (Ia) or (Ia'):

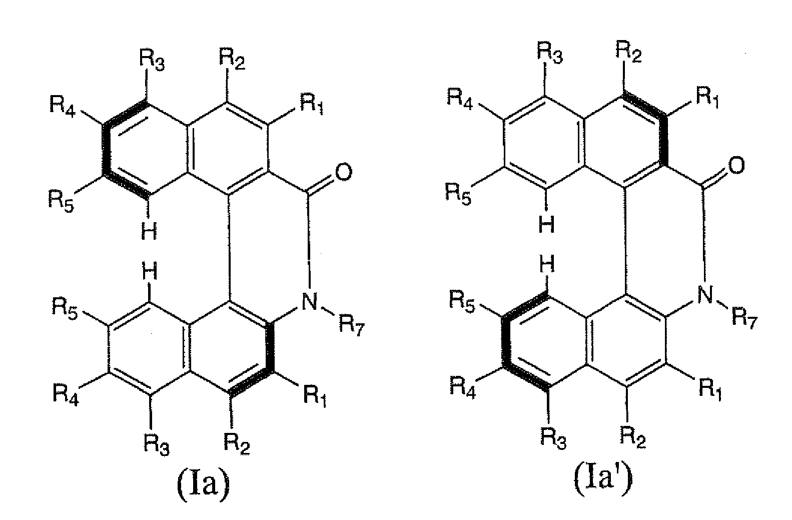

wherein R₁, R₂, R₃, R₄, and R₅ may be the same or different, and represent hydrogen, alkyl, cycloalkyl, aryl, heteroaryl, alkenyl, alkynyl, cyano, alkoxy, hydroxy, acyloxy, CONH₂, monoalkylaminocarbonyl, dialkylaminocarbonyl, nitro, amino, monoalkylamino, dialkylamino, alkanoylamino, azido, carboxy (COOH), alkoxycarbonyl, or acyl; two adjacent groups among R₁, R₂, R₃, R₄, and R₅ may be joined together with the carbon atoms to which they are attached to form a 5- or 6-membered saturated or unsaturated ring structure or structures, and these ring structures may be substituted; and R₇ represents hydrogen, alkyl, cycloalkyl, aryl, heteroaryl, acyl, or alkoxycarbonyl.

The helicene derivative, the axially chiral amino acid, amine, or amino alcohol derivative of the present invention can be used as asymmetric catalysts, asymmetric molecule recognition elements, and fundamental skeletons for organic materials having specific optical properties. Further, perylene is also expected to be used as an organic EL material and like organic optical materials, a sensor element, and a fluorescent reagent.

The production method of the present invention comprises three stages: a first stage in which the compound represented by the above Formula (IV) is reacted in the presence of a Pd catalyst; a second stage in which the compound represented by the above Formula (I) or (I') is reacted in the presence of an alkali metal hydroxide; and a third stage in which the compound represented by the above Formula (Ia) or (Ia') is reacted in the presence of a sulfonic acid.

The first stage is the homocoupling reaction of Formula (IV) for forming a helicene derivative. Aprotic polar solvents such as DMF, DMSO, and the like are usable as solvents.

The concentration of the compound represented by the above Formula (IV) in the solvent may be in the range from relatively low (0.01 M to 1.0 M) to relatively high. Pd₂(dba)₃, Pd(OAc)₂, PdCl₂, or the like is usable as a catalyst. Such a catalyst is added in an amount of 0.001 to 0.1 equivalents of the compound represented by the above Formula (IV) to carry out the reaction. The reaction time is about 1 to 24 hours.

As the base, K₂CO₃, Cs₂CO₃, or the like may be used in an equivalent amount. The concentration of the base in the solvent is usually 0.001 to 1.0 M, preferably 0.01 to 0.1 M. The reaction temperature is usually 20°C to 150°C, preferably 70°C to 100°C.

The reaction in this stage produces the helicene derivative represented by the above Formula (I) or (I').

The second stage is a reaction to give the axially chiral amino acid derivative of Formula (II) or (II') by cleaving the amide bond in the above Formula (I) or (I'). This reaction can be carried out under the conditions that a metal hydroxide such as LiOH, NaOH, KOH or the like is added to the aprotic polar solvent such as DMSO. The concentration of the compound represented by the above Formula (I) or (I') in the solvent is preferably 0.01 to 1.0 M. A metal alkoxide in an amount of about 1 to 10 equivalents of the compound represented by the above Formula (I) or (I') is added thereto. The reaction temperature is usually 20°C to 150°C.

The reaction in this stage produces the axially chiral amino acid derivative represented by the above Formula (II) or (II') . At this amine stage, an R₈ group may be introduced into the amino group, and the COOH group can be reduced by an appropriate reducing reagent (NaBH₄, LiAlH₄, etc.) to be converted to an alcohol (CH₂OH).

The COOH group can be converted to CZ₂OH (Z is as defined above other than hydrogen) by reacting with Z-MgBr (Grignard reagent), a lithium reagent represented by Z-Li, or the like. Alternatively, COOH can be converted to an ester (COOZ) and an amide (CONZ₂) by dehydration-condensation with Z-OH and NHZ₂, respectively. When the amide is CONH₂, it can be converted to CN by dehydration, and subsequently to tetrazole.

Additionally, the amino group can also be protected with a protecting group such as Boc, Cbz, Alloc, Fmoc, or the like, and R₈ can also be introduced into the amino group.

The third stage is a reaction to obtain the perylene derivative represented by the above Formula (III) by intramolecular carbon-carbon bond formation by a sulfonic acid in the above Formula (Ia) or (Ia'). Trifluoromethanesulfonic acid or the like is used as the sulfonic acid to carry out the reaction. The concentration of Formula (Ia) or (Ia') in the acid is preferably 0.01 to 1.0 M. The reaction temperature is usually 0°C to 30°C.

The reaction in this stage produces the perylene derivative represented by the above Formula (III).

Although the production method of the present invention is characterized in comprising the above-described reactions in three stages as the main reactions, a known reaction may be suitably added before and/or after these reactions. Examples of such a reaction include (A) amide bond forming reaction, (B) amide or amino deprotection reaction, and the like. These reactions are not the characteristic features of the present invention, and may be carried out according to a general method; non-limiting examples of such general methods are shown below.

(A) Usually, an amide bond forming reaction is carried out as follows: a carboxylic acid is reacted with an amine in an aprotic polar solvent, using a condensing agent (DCC, EDCI, etc.); or a carboxylic acid is converted to an acid halide, and the acid halide is then reacted with an amine in an aprotic polar solvent in the presence of a base.
(B) An amide or amino deprotection reaction varies depending on a protecting group; however, it is usually carried out as follows: when the protecting group is p-methoxyphenyl or p-methoxybenzyl, the reaction is carried out under oxidizing conditions using CAN, DDQ, hypervalent iodine reagent, etc.; and when the protecting group is benzyl or the like, the reaction is carried out under catalytic hydrogenation conditions (for example, in the presence of a hydrogen gas to which a Pd catalyst is added).

### Examples

The present invention is explained in detail below with reference to Examples. However, the scope of the invention is not limited to these Examples.

### Example 1

### Synthesis of N-benzyl-1-bromo-2-naphthamide (2)

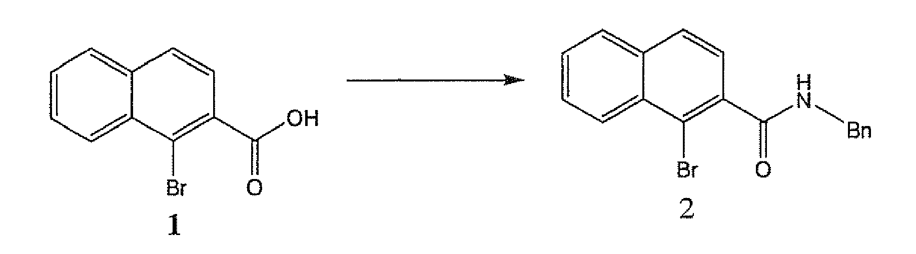

SOCl₂ (0.090 mL, 1.31 mmol) and a catalytic amount of DMF were added to a toluene solution (4.5 mL) of 1-bromo-2-naphthoic acid (1) (300 mg, 1.19 mmol) in an Ar atmosphere, followed by stirring at 80°C for 2 hours. After distilling off the reaction liquid under reduced pressure, the residue was dissolved in CH₂Cl₂ (4.0 mL). Benzylamine (0.16 mL, 1.43 mmol) and Et₃N (0.50 mL, 3.58 mmol) were added to the dissolution at 0°C, and the mixture was then stirred at room temperature for 15 hours. After adding water, the mixture was subjected to extraction using CH₂Cl₂ three times, washed with a saturated sodium hydrogencarbonate aqueous solution, 2N aq. HCl, and a saturated sodium chloride solution, dried over MgSO₄, and then condensed under reduced pressure. Colorless crystal N-benzyl-1-bromo-2-naphthamide (2) (362 mg, 89%) was thus obtained.
¹H NMR (270 MHz, CDCl₃) : 58.33 (d, J = 8.1 Hz, 1H), 7.90-7.80 (m, 2H), 7.70-7.20 (m, 8H), 6.26 (brs, 1H), 4.71 (d, J = 4.8 Hz, 2H).

### Synthesis of 3-benzyldibenzo[a,k]phenanthridin-4(3H)-one (3)

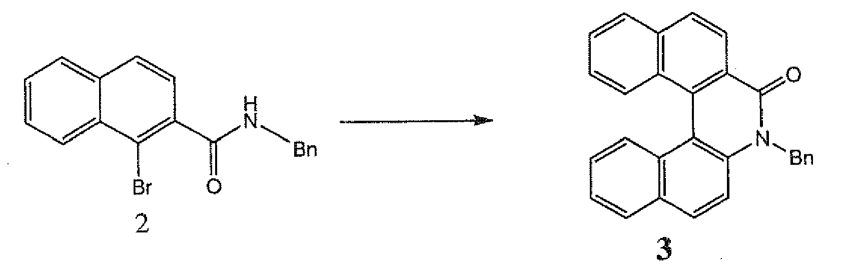

Pd₂ (dba)₃ (19 mg, 0.021 mmol) , K₂CO₃ (48 mg, 0.35mmol), and DMF (3.0 mL) were added to N-benzyl-1-bromo-2-naphthamide (2) (124 mg, 0.35 mmol) in an Ar atmosphere, and the mixture was stirred at room temperature for 16 hours. After adding a saturated ammonium chloride aqueous solution, the mixture was subjected to extraction using EtOAc, dried over anhydrous MgSO₄, and then condensed under reduced pressure. Subsequently, the resulting condensate was purified using silica gel column chromatography (n-hexane:EtOAc = 5:1), obtaining yellow crystal 3-benzyldibenzo[a,k]phenanthridin-4(3H)-one (3) (66 mg, 98%). ¹H NMR (500 MHz, CDCl₃): δ8.60 (d, J = 8.6 Hz, 1H) , 8.10-8.02 (m, 2H), 8.00 (d, J = 8.0 Hz, 1H), 7.93 (d, J = 8.6 Hz, 1H), 7.90-7.80 (m, 2H), 7.65-7.58 (m, 1H), 7.55 (d, J = 9.2 Hz, 1H), 7.45-7.37 (m, 1H), 7.35-7.18 (m, 7H), 5.89 (brs, 1H), 5.63 (brs, 1H).

### Synthesis of 2'-(benzylamino)-1,1'-binaphthyl-2-carboxylic acid (4)

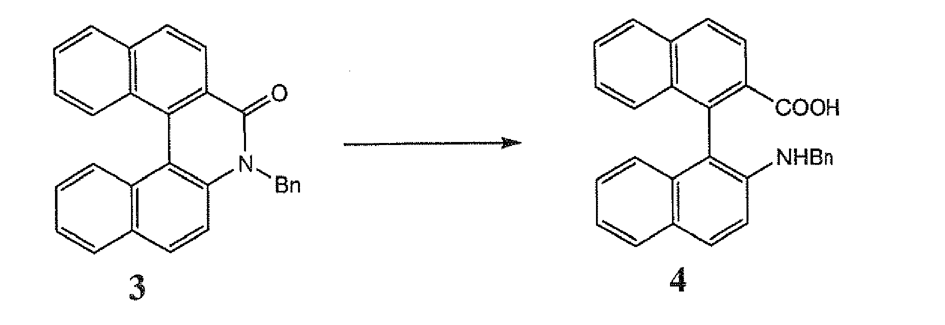

NaOH (10 mg, 0.26 mmol) and DMSO (1.0 mL) were added to 3-benzyldibenzo[a,k]phenanthridin-4(3H)-one (3) (10 mg, 0.026 mmol) at room temperature, and the mixture was stirred at room temperature for 4 hours, then at 50°C for 17 hours, and at 80°C for 1 hour. After adding water (7.0 mL), the reaction liquid was neutralized with 1N aq. HCl, subjected to extraction using EtOAc, washed with a saturated sodium chloride solution, dried over anhydrous MgSO₄, and then condensed under reduced pressure. Subsequently, the resulting condensate was purified using silica gel column chromatography (n-hexane:EtOAc = 1:1), obtaining 2'-(benzylamino)-1,1'-binaphthyl-2-carboxylic acid (4) (9.1 mg, 87%). ¹H NMR (270 MHz, CDCl₃): δ7.51-7.53 (m, 5H), 7.24-7.42 (m, 15H), 6.70 (s, 2H), 5.07 (s, 4H), 5.03 (s, 2H), 4.52 (s, 2H).

### Synthesis of 1-benzylphenanthro[1,10,9,8-klmna]phenanthridin-2(1H)-one (5)

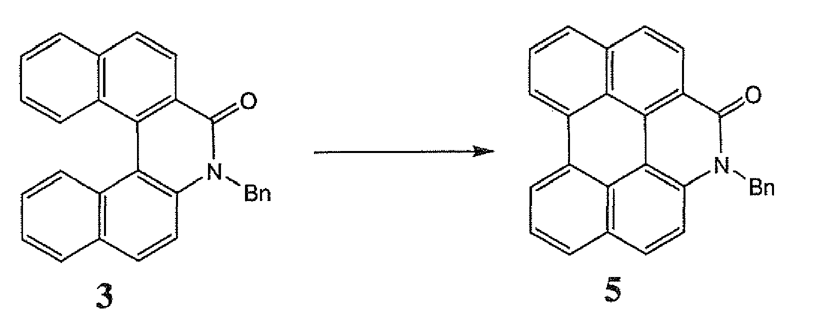

Trifluoromethanesulfonic acid (0.5 mL) was added to 3-benzyldibenzo[a,k]phenanthridin-4(3H)-one (3) (10 mg, 0.024 mmol) at room temperature, and the mixture was stirred at room temperature for 4 hours. The reaction liquid was added to water (10 mL), and the mixture was then subjected to extraction using EtOAc. The resulting organic layer was washed with a saturated sodium hydrogencarbonate aqueous solution and a saturated sodium chloride solution, dried over anhydrous Na₂SO₄, and then condensed under reduced pressure. Subsequently, the resulting condensate was purified using preparative TLC (SiO₂, n-hexane:EtOAc = 2:1), obtaining 1-benzylphenanthro[1,10,9,8-klmna]phenanthridin-2(1H)-one (5) (2.8 mg, 30%).
¹H NMR (270 MHz, CDCl₃): δ8. 70-7.60 (m, 10H), 7.40-7.10 (m, 5H), 6.10-5.85 (br s, 2H).

### Example 2

### Synthesis of 1-bromo-N-(4-methoxybenzyl)-2-naphthamide (6)

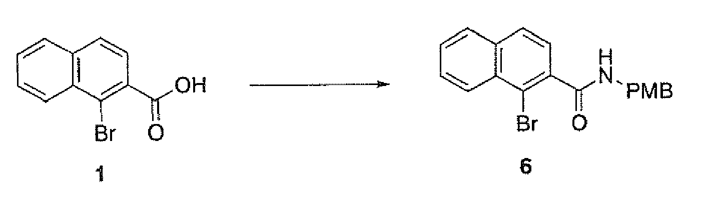

SOCl₂ (0.31 mL, 4.38 mmol) and a catalytic amount of DMF were added to a toluene solution (10 mL) of 1-bromo-2-naphthoic acid (1) (1.00 g, 3.98 mmol) in an Ar atmosphere, and the mixture was stirred at 75°C for 4 hours. After distilling the reaction liquid off under reduced pressure, the residue was dissolved in CH₂Cl₂ (4.0 mL). p-Methoxybenzylamine (0.62 mL, 4.78 mmol) and Et₃N (1.68 mL, 11.9 mmol) were added to the solution at 0°C, and the mixture was stirred at room temperature for 2 hours. After adding a saturated sodium hydrogencarbonate aqueous solution, the mixture was subjected to extraction using AcOEt 4 times, washed with a saturated ammonium chloride aqueous solution and a saturated sodium chloride solution, dried over MgSO₄, and then condensed under reduced pressure. The crude product thus obtained was recrystallized using toluene-hexane, and the resulting crystal was washed with a small amount of methanol, obtaining the target product in the form of colorless crystal 1-bromo-N-(4-methoxybenzyl)-2-naphthamide (6) (1.16 g, 79%).
Colorless needles (n-hexane-toluene). m.p. 155°C; ¹H NMR (400 MHz, CDCl₃) δ 3.79 (s, 3H), 4.60 (d, J = 5.5 Hz, 2H), 6.28 (br s, 1H), 6.87 (d, J = 8.7 Hz, 2H), 7.32 (d, J = 8.7 Hz, 2H), 7.46 (d, J = 8.7 Hz, 1H), 7.53-7.65 (m, 2H), 7.78 (t, J = 8.7 Hz, 2H), 8.30 (d, J = 8.2 Hz, 1H) ; ¹³C NMR (100 MHz, CDCl₃) δ 43.8, 55.3, 114.0, 119.8, 125.0, 127.6, 127.8, 128.1, 128.2, 129.4, 129.6, 131.8, 134.5, 136.2, 159.1, 168.4; IR (KBr) 1635, 2833, 2917, 3068, 3280, 3431 cm⁻¹; MS (FAB) m/z 370 (M+H)⁺, 392 (M+Na)⁺; HRMS (FAB) m/z calcd for C₁₉H₁₇NO₂⁷⁹Br (M+H)⁺ 370.0442, found 370.0450, calcd for C₁₉H₁₇NO₂⁸¹Br (M+H)⁺ 372.0422, found 372.0416.

### Synthesis of 3-p-methoxybenzyldibenzo[a,k]phenanthridin-4(3H) -one (7)

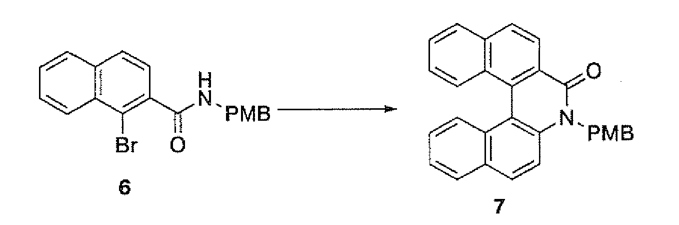

Pd₂ (dba) ₃ (198 mg, 0.22 mmol) , K₂CO₃ (6.57 g, 47.5 mmol), and DMF (80 mL) were added to 1-bromo-N-(4-methoxybenzyl)-2-naphthamide (6) (16.0 g, 43.2 mmol) in an Ar atmosphere, followed by stirring at 100°C for 36 hours. After adding water, the mixture was subjected to extraction using chloroform, dried over anhydrous Na₂SO₄, and then condensed under reduced pressure. The crude product thus obtained was purified by recrystallization. The filtrate after recrystallization was purified using silica gel column chromatography (n-hexane:chloroform:EtOAc = 5:3:1), obtaining yellow crystal 3-benzyldibenzo[a,k]phenanthridin-4(3H)-one (7) (8.61 g, 99%).
Light yellow needles (n-hexane-AcOEt) ; m.p. 229 °C; ¹H NMR (400 MHz, CDCl₃) δ 3.74 (s, 3H), 5.57 (br d, J = 13.8Hz, 1H), 5.82 (br d, 1H) , 6.82 (d, J = 8.7 Hz, 2H), 7.20-7.35 (m, 4H), 7.39-7.44 (m, 1H), 7.56-7.64 (m, 2H), 7.81-7.94 (m, 3H), 7.96-8.09 (m, 3H), 8.50 (d, J = 8.7 Hz, 1H); ¹³C NMR (100 MHz, CDCl₃) δ 46.3, 55.2, 113.5, 114.2, 115.4, 123.7, 124.8, 124.9, 125.3, 125.7, 127.7, 127.8, 128.0, 128.1, 128.5, 128.7, 129.1, 129.2, 129.5, 130.4, 132.8, 135.4, 136.5, 158.7, 162.2; IR (KBr) 1690, 2956, 3057, 3446 cm⁻¹; MS (FAB) m/z 416 (M+H)⁺, 438 (M+Na); HRMS (FAB) m/z calcd for C₂₉H₂₂NO₂ (M+H)⁺ 416.1650, found 416.1649. Dibenzo[a,k]phenanthridin-4(3H)-one (8)

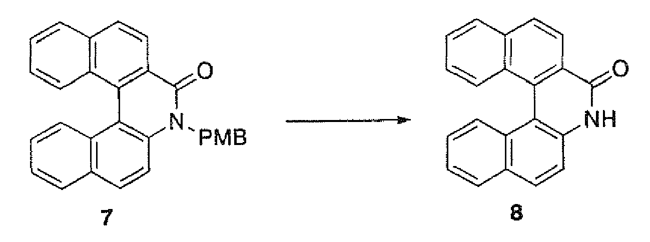

TFA (3.0 mL) was added to 3-benzyldibenzo [a, k]phenanthridin-4 (3H) -one (7) (295 mg, 0.71 mmol), and the mixture was stirred at room temperature for 38 hours. The reaction liquid was added to an ice-cooled saturated sodium hydrogencarbonate aqueous solution dropwise, and the produced pale yellow solid was separated by filtration. The separated solid was washed with water, and further washed with acetone and diethyl ether, obtaining pale yellow powder dibenzo[a,k]phenanthridin-4(3H)-one (8) (211 mg, 100%). ¹H NMR (400 MHz, CDCl₃) : 57.30-7.40 (m, 2H), 7.40-7.50 (m, 1H), 7.60-7.70 (m, 1H), 7.92 (d, J = 8.0 Hz, 1H), 7.95-8.10 (m, 4H), 8.21 (d, J = 8.5 Hz, 1H), 8.51 (d, J = 7.9 Hz, 1H), 9.13 (br s, 1H); IR (KBr) 1620, 1657, 2833, 2878, 3011, 3152, 3033, 3450 cm⁻¹; MS (FAB) m/z 296 (M+H)⁺, 318 (M+Na)⁺; HRMS (FAB) m/z calcd for C₂₁H₁₄NO (M+H)⁺295.1075, found 295.1076.

### Synthesis of tert-butyl 4-oxodibenzo[a,k]phenanthridine-3(4H)-carboxylate (9)

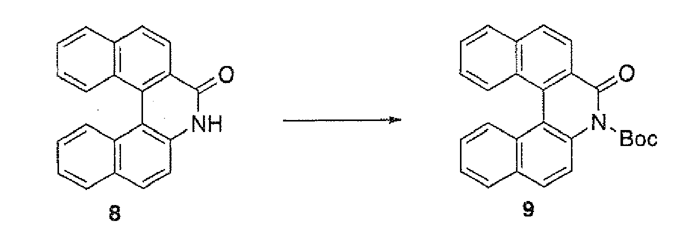

A THF solution (30 mL) of dibenzo[a,k]phenanthridin-4(3H)-one (8) (726 mg, 2.46 mmol) was cooled to -78°C in an Ar atmosphere, n-BuLi (1.59 M in hexane, 3.71 mL, 5.90 mmol) was added thereto, and the mixture was stirred at 0°C for 1.5 hours. A THF solution (20 mL) of Boc₂O (1.40 g, 6.40 mmol) was added to the resulting mixture at 0°C, and the result was stirred at the same temperature for 8 hours. After adding a saturated ammonium chloride aqueous solution, the mixture was subjected to extraction using ethyl acetate, dried over anhydrous Na₂SO₄, and then condensed under reduced pressure. The crude product thus obtained was recrystallized using toluene, obtaining yellow crystal tert-butyl 4-oxodibenzo[a,k]phenanthridine-3(4H)-carboxylate (9) (762 mg, 78%).
Light yellow needles (n-hexane-AcOEt); 300°C (sublimed); ¹H NMR (400 MHz, CDCl₃) 5 1.79 (s, 9H), 7.25-7.35 (m, 3H), 7.40 (d, J = 8.7 Hz, 1H), 7.43-7.47 (m, 1H), 7.60-7.64 (m, 1H), 7.88-7.92 (m, 2H), 7.92-8.08 (m, 4H), 8.47 (d, J = 8.7 Hz, 1H); ¹³C NMR (100 MHz, CDCl₃) δ 27.6, 86.8, 112.6, 114.2, 122.9, 125.0, 125.1, 125.4, 125.7, 127.6, 128.0, 128.1, 128.4, 128.9, 129.1 129.2, 130.2, 130.4, 130.9, 133.5, 133.6, 135.8, 151.2, 160.2; IR (KBr) 1659, 1765, 2977, 3435 cm⁻¹; MS (FAB) m/z 395 (M)⁺, 396 (M+H)⁺, 418 (M+Na)⁺; HRMS (FAB) m/z calcd for C₂₆H₂₁NO₃ (M)⁺ 395.1522, found 395.1516.

### Synthesis of (S)-((S)-1-phenylethyl) 2-(tert-butoxycarbonylamino)-1,1'-binaphthyl-2'-carboxylate (10)

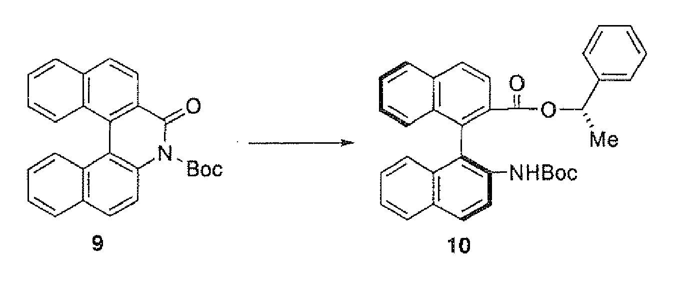

A THF solution (70 mL) of tert-butyl 4-oxodibenzo[a,k]phenanthridine-3(4H)-carboxylate (9) (20 mg, 0.051 mmol) was cooled to -78°C in an N₂ atmosphere, and a separately prepared alkoxide of phenylethyl alcohol (prepared by pouring THF solution:60% NaH (301 mg, 7.52 mmol) into a pear shaped flask, replacing the atmosphere therein with N₂, adding THF (20 ml) thereto, and then adding S-phenylethyl alcohol (0.9 mL, 7.3 mmol) dropwise) was added thereto dropwise. After the reaction was completed, the reaction was stopped with a saturated ammonium chloride aqueous solution, followed by extraction using ethyl acetate (5 mL × 4). The resulting extract was washed with a saturated sodium chloride solution, and dried over Na₂SO₄, and the solvent was distilled off under reduced pressure. The crude product was purified using column chromatography (H:EA = 10:1), obtaining 3.45 g (100%) of the target product. Diastereomer was separated from the resulting compound by recrystallization (n-hexane:ethyl acetate = 20:1), obtaining (S)-((S)-1-phenylethyl) 2-(tert-butoxycarbonylamino)-1,1'-binaphthyl-2'-carboxylate (10) (1.36 g, 40%).
Colorless prisms (n-hexane-AcOEt); m.p. 147°C; [α]_{D}²⁰ = +70 (c 1.00, CHCl₃); ¹H NMR (400 MHz, CDCl₃) δ 0.96 (d, J = 6.9 Hz, 3H), 1.35 (s, 9H), 5.68 (q, J = 6.4 Hz, 1H), 5.99 (br s, 1H) , 6.49 (d, J = 8.7 Hz, 2H), 6.82 (d, J = 8.7 Hz, 1H), 7.00 (t, J = 7.8 Hz, 2H), 7.05-7.11 (m, 1H), 7.12-7.18 (m, 1H), 7.22-7.26 (m, 1H), 7.27-7.33 (m, 1H), 7.35-7.41 (m, 1H), 7.53-7.59 (m, 1H), 7.90 (d, J = 8.3 Hz, 1H), 7.94-7.99 (m, 2H), 8.06 (d, J = 8.7 Hz, 1H), 8.15 (d, J = 8.7 Hz, 1H), 8.35 (br d, J = 8.2 Hz, 1H); ¹³C NMR (100 MHz, CDCl₃) δ 21.7, 28.1, 73.5, 80.5, 124.4, 125.5, 125.7, 126.4, 126.5, 127.2, 127.4, 127.8, 128.05, 128.13, 128.2, 128.5, 129.1, 130.2, 130.3, 132.5, 133.3, 134.3, 134.5, 135.3, 141.1, 152.9, 166.8; IR (KBr) 1705, 1752, 1873, 2933, 2980, 3064, 3927 cm⁻¹; MS (FAB) m/z 517 (M)⁺, 518 (M+H)⁺, 540 (M+Na)⁺; HRMS (FAB) m/z calcd for C₃₄H₃₁NO₄ (M)⁺ 517.2253, found 517.2251.

### Synthesis of (S)-2'-(tert-butoxycarbonylamino)-1,1'-binaphthyl-2-carboxylic acid (11)

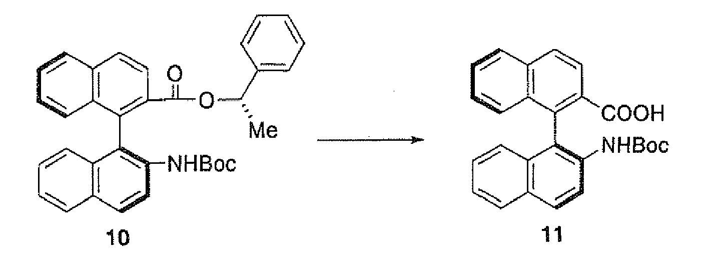

A MeOH solution (2 mL) of (S)-((S)-1-phenylethyl) 2-(tert-butoxycarbonylamino)-1,1'-binaphthyl-2'-carboxylate (10) (50 mg, 0.097 mmol) and Pd(OH)₂/C (5 mg) was subjected to replacement with H₂, and then stirred at room temperature for 1.5 hours. After the reaction was completed, Pd(OH)₂/C was removed by filtration, and the filtrate was distilled off under reduced pressure to obtain (S)-2'-(tert-butoxycarbonylamino)-1,1'-binaphthyl-2-carboxylic acid (11) (38.2 mg, 95%).
Colorless prisms (n-hexane-AcOEt) ; 165°C (decomp.); [α]_{D}²⁰ = -133 (c 0.6, CHCl₃) ; ¹H NMR (400 MHz, CDCl₃) δ 1.24 (s, 9H), 6.22 (br s, 1H), 6.81 (d, J = 8.7 Hz, 1H), 7.10-7.18 (m, 2H), 7.22-7.35 (m, 2H), 7.53 (t, J = 7.2 Hz, 1H), 7.84 (d, J = 8.2 Hz, 1H), 7.93 (t, J = 8.2 Hz, 2H), 7.99-8.07 (m, 2H), 10.21 (br s, 1H); ¹³C NMR (100 MHz, CDCl₃) δ 28.0, 80.7, 124.8, 125.1, 126.1, 126.4, 127.2, 127.3, 127.8, 128.0, 128.2, 128.7, 128.9, 129.2, 130.8, 132.3, 132.9, 133.8, 135.4, 135.9, 171.2; IR (KBr) 1698, 1725, 2618, 2928, 2975, 3064, 3064, 3426 cm⁻¹; MS (FAB) m/z 413 (M)⁺, 414 (M+H)⁺, 436 (M+Na)⁺; HRMS (FAB) m/z calcd for C₂₆H₂₃NO₄ (M+H)⁺ 413.1627, found 413.1626.

### Synthesis of (S)-2'-amino-1,1'-binaphthyl-2-carboxylic acid (12)

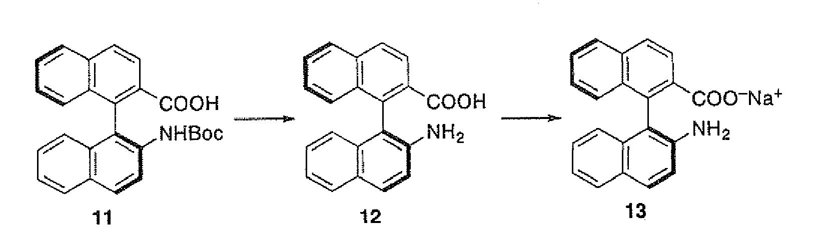

TFA (0.2 ml) was added to a CH₂Cl₂ solution (2 mL) of (S) -2'-(tert-butoxycarbonylamino)-1,1'-binaphthyl-2-carboxylic acid (11) (5.3 mg, 0.013 mmol), and the mixture was stirred for 45 minutes. After the reaction was completed, the reaction was stopped with 2N NaOH, and it was confirmed that the pH was about 15 using a pH test paper. The water layer was washed with diethyl ether (3mL×3). After confirming that there was no product in the organic layer using TLC, the water layer was neutrizied to pH 7, subjected to extraction with chloroform (5 mL×3), and dried over Na₂SO₄. Thereafter, the solvent was distilled off to obtain (S)-2'-amino-1,1'-binaphthyl-2-carboxylic acid (12) (11 mg, 76%). (S)-2'-amino-1,1'-binaphthyl-2-carboxylic acid (12): [α]_{D}²⁰ = -23 (c 0.32, 2M MeONa in MeOH)
Furthermore, 3 to 5 equivalent weight of NaOH was added to the resulting product and dissolved in methanol. The solvent was distilled off to obtain (S)-2'-amino-1,1'-binaphthyl-2-carboxylic acid sodium salt (13).
(S)-2'-amino-1,1'-binaphthyl-2-carboxylic acid sodium salt (13): ¹H NMR (400 MHz, CD₃OD) δ 6.78 (d, J = 8.3 Hz, 1H), 6.93-7.04 (m, 3H), 7.06-7.14 (m, 2H), 7.30-7.37 (m, 1H), 7.59-7.65 (m, 2H), 7.66 (dd, J = 1.4, J = 8.7 Hz, 1H), 7.82 (d, J = 8.7 Hz, 1H) 7.88 (d, J = 8.7 Hz, 1H); ¹³C NMR (100 MHz, CD₃OD) δ117.5, 118.5, 121.5, 124.7, 124.9, 125.5, 125.7, 126.0, 126.3, 127.2, 127.6, 127.7, 128.2, 128.3, 130.6, 132.7, 133.9, 134.5, 139.4, 142.8, 175.8; IR (KBr) 1685, 3334 cm⁻¹, 3410 cm⁻¹; MS (FAB) m/z 336 (M+H)⁺, 358 (M+Na)⁺; HRMS (FAB) m/z calcd for C₂₁H₁₅O₂NNa (M+H)⁺336.1000, found 336.0993.

### Synthesis of (S)-tert-butyl 2'-(hydroxymethyl)-1,1'-binaphthyl-2-ylcarbamate (14)

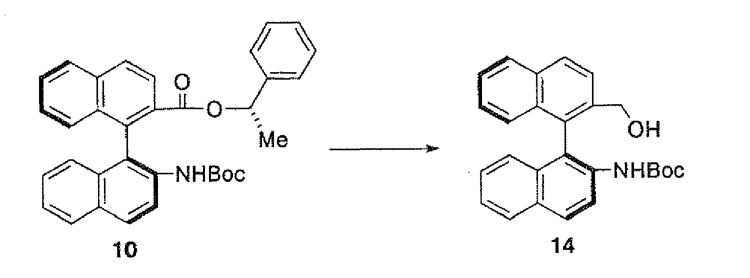

A THF solution (1 mL) of (S)-((S)-1-phenylethyl) 2-(tert-butoxycarbonylamino)-1,1'-binaphthyl-2'-carboxylate (10) (21.4 mg, 0.041 mmol) was cooled to 0°C in a N₂ atmosphere. After adding a THF solution of LiBH₄ (3 M, THF solution) (0.069 mL, 0.21 mmol) dropwise at 0°C, the mixture was warmed to room temperature and then stirred for 24 hours. After the reaction was completed, the reaction was stopped with water, and extraction using ethyl acetate (5 mL × 3) was performed. The resulting extract was washed with a saturated NaCl aqueous solution and dried over Na₂SO₄. Thereafter, the solvent was distilled off under reduced pressure. The crude product was purified with preparative TLC (hexane:ethyl acetate = 10:1), obtaining (S)-tert-butyl 2'-(hydroxymethyl)-1,1'-binaphthyl-2-ylcarbamate (14) (8.9 mg, 54%).
Colorless needles (CHCl₃) ; m.p. 132°C; [α]_{D}²⁰ = -66 (c 2.57, CHCl₃); ¹H NMR (400 MHz, CDCl₃) δ 1.34 (s, 9H), 4.37 (S, 2H), 6.12 (s, 1H), 6.88 (d, J = 8.2 Hz, 1H), 7.13 (d, J = 8.2 Hz, 1H), 7.15-7.22 (m, 1H), 7.24-7.30 (m, 1H), 7.33-7.40 (m, 1H), 7.46-7.53 (m, 1H), 7.83-7.90 (m, 2H), 7.94-7.99 (m, 2H), 8.06 (d, J = 8.7 Hz, 1H), 8.35 (d, J = 9.2 Hz, 1H) ; ¹³C NMR (100 MHz, CDCl₃) δ 28.1, 63.2, 80.7, 120.5, 122.3, 124.7, 125.0, 125.7, 126.2, 126.8, 126.9, 128.1, 128.2, 129.0, 129.3, 130.4, 132.4, 132.9, 133.4, 134.6, 138.1, 153.2; IR (KBr) 1618, 2873, 2928, 3051, 3376 cm⁻¹; MS (FAB) m/z 399 (M)⁺, 400 (M+H)⁺, 422 (M+Na) ⁺; HRMS (FAB) m/z calcd for C₂₆H₂₅NO₃ (M)⁺ 399.1835, found 399.1827.

### Synthesis of (S)-tert-butyl 2'-(hydroxydiphenylmethyl)-1,1'-binaphthyl-2-ylcarbamate (15)

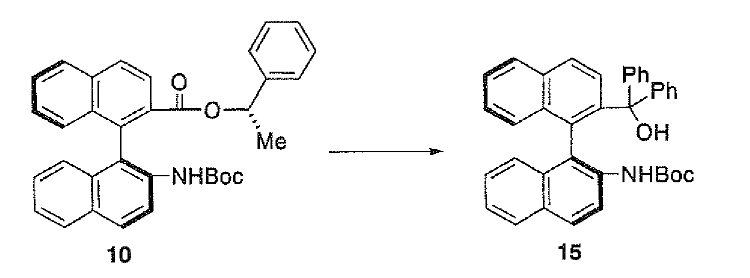

A THF solution (15 ml) of (S)-((S)-1-phenylethyl) 2-(tert-butoxycarbonylamino)-1,1'-binaphthyl-2'-carboxylate (10) (300 mg, 0.57 mmol) was cooled to -78 °C in a N₂ atmosphere. After adding PhLi (1.08 M, cyclohexane solution) (5.5 mL, 5.7 mmol) thereto dropwise, the temperature was gradually cooled to room temperature, and the mixture was stirred for 6 hours. After the reaction was completed, the reaction was stopped with a saturated ammonium chloride aqueous solution, extraction using ethyl acetate (5 mL × 3) was performed, and the extract was washed with a saturated NaCl aqueous solution and then dried over Na₂SO₄. Thereafter, solvent was distilled off under reduced pressure. The crude product was purified using silica gel column chromatography (hexane:ethyl acetate = 13:1), and preparative liquid chromatography was further conducted, obtaining (S)-tert-butyl 2'-(hydroxydiphenylmethyl)-1,1'-binaphthyl-2-ylcarbamate (15) (258 mg, 82%).
Colorless prisms (hexane : ethyl acetate); m.p. 103°C; [α]_{D}²⁰ = -13 (c 1.8, CHCl₃) ; ¹H NMR (400 MHz, CDCl₃) δ 1.34 (s, 9H), 2.78 (s, 1H), 5.86 (s, 1H), 6.74 (d, J = 8.2 Hz, 1H), 6.90 (d, J = 8.7 Hz, 1H), 6.94-7.00 (m, 1H), 7.03-7.09 (m, 2H), 7.10-7.33 (m, 10H), 7.42-7.48 (m, 1H), 7.78 (d, J = 8.2 Hz, 1H), 7.83-7.95 (m, 3H), 8.30 (brs, 1H); ¹³C NMR (100 MHz, CDCl₃) 5 28.1, 80.3, 84.0, 124.4, 125.7, 125.9, 126.2, 126.6, 127.07, 127.09, 127.4, 127.5, 127.6, 127.7, 127.8, 128.2, 129.1, 129.2, 129.9, 130.3, 132.7, 132.9, 133.5, 134.9, 143.9, 146.1, 146.6, 152.6; IR (KBr) 1729, 2928, 2977, 3060, 3416 cm⁻¹; MS (FAB) m/z 551 (M)⁺, 552 (M+H)⁺, 574 (M+Na)⁺; HRMS (FAB) m/z calcd for C₃₈H₃₃NO₃ (M)⁺ 551.2460, found 551.2465.

### Synthesis of (S)-(2'-amino-1,1'-binaphthyl-2-yl)methanol (16)

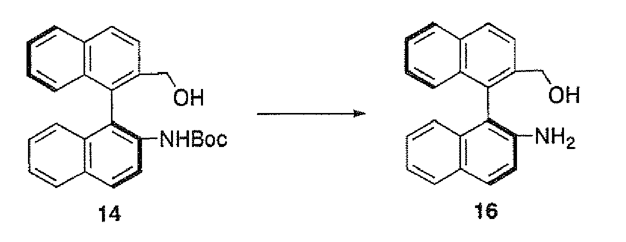

TFA (0.2 mL) was added to a CH₂Cl₂ solution (2.0 mL) of (S)-tert-butyl 2'-(hydroxymethyl)-1,1'-binaphthyl-2-ylcarbamate (14) (5.3 mg, 0.013 mmol), and the mixture was stirred at room temperature for 1.5 hours. After the reaction was completed, the reaction was stopped using a saturated NaHCO₃ aqueous solution, followed by extraction using ethyl acetate (3 mL × 3). The extract was washed with a saturated NaCl aqueous solution, dried over Na₂SO₄, and then the solvent was distilled off under reduced pressure. The resulting crude product was purified using preparative TLC (hexane:ethyl acetate = 7:1), obtaining (S)-(2'-ainino-1,1'-binaphthyl-2-yl)methanol (16) (10.2 mg, 83%). Colorless needles (CHCl₃); m.p. 72°C; [α]_{D}²⁰ = -42 (c 0.5, THF); ¹H NMR (400 MHz, CDCl₃) δ 3.11 (br s, 3H), 4.54 (S, 2H), 6.84 (d, J = 8.2 Hz, 1H), 7.07-7.18 (m, 2H), 7.21-7.30 (m, 3H), 7.47 (t, J = 7.8 Hz, 1H), 7.77-7.853 (m, 3H), 7.93 (d, J = 7.8 Hz, 1H), 8.00 (d, J = 8.7 Hz, 1H); ¹³C NMR (100 MHz, CDCl₃) δ 64.3, 116.3, 118.2, 122.8, 124.1, 125.9, 126.1, 126.6, 126.8, 127.6, 128.1, 128.2, 128.4, 128.9, 129.4, 132.0, 132.5, 133.7, 133.9, 138.5, 141.4; IR (KBr) 1501, 1726, 2977, 3410 cm⁻¹ ; MS (FAB) m/z 299 (M)⁺, 300 (M+H)⁺; HRMS (FAB) m/z calcd for C₂₁H₁₇NO (M+H)⁺ 299.1310, found 399.1317.

### Synthesis of (S)-(2'-amino-1,1'-binaphthyl-2-yl)diphenylmethanol (17)

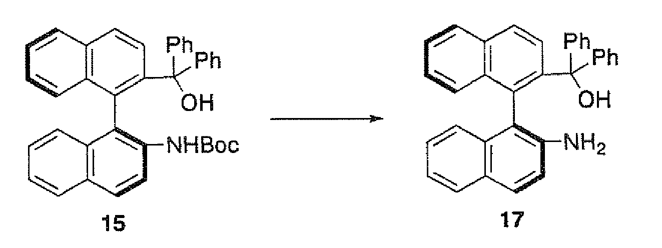

t-BuONa (30 mg, 0.295 mmol) was added to a THF solution (6.0 mL) of (S)-tert-butyl 2'-(hydroxydiphenylmethyl)-1,1'-binaphthyl-2-ylcarbamate (15) (32.6 mg, 0.059 mmol) in a N₂ atmosphere, and the mixture was heated to 66°C and then stirred for 4.5 days. After the reaction was completed, the reaction was stopped using a saturated ammonium chloride aqueous solution, and extraction using ethyl acetate (5 mL x 3) was conducted. The resulting extract was washed with a saturated NaCl aqueous solution and dried over Na₂SO₄; the solvent was then distilled off under reduced pressure, and purified using silica gel column chromatography (hexane:ethyl acetate = 15:1), obtaining (S)-(2'-amino-1,1'-binaphthyl-2-yl)diphenylmethanol (17) (16.1 mg, 61%). Colorless needles (CHCl₃); m.p. 113°C; [α]_{D}²⁰ = +29 (c 0.5, CHCl₃); ¹H NMR (400 MHz, CDCl₃) δ 6.73 (d, J = 8.7 Hz, 1H), 6.93 (d, J = 8.7 Hz, 1H), 6.95-7.01 (m, 1H), 7.01-7.07 (m, 3H), 7.08-7.17 (m, 1H), 7.18-7.33 (m, 8H), 7.44 (t, J = 7.8 Hz 1H), 7.67 (d, J = 8.2 Hz, 1H), 7.73 (d, J = 8.7 Hz, 1H), 7.82 (d, J= 8.6 Hz, 1H), 7.88 (d, J = 7.8 Hz, 1H) ; ¹³C NMR (100 MHz, CDCl₃) δ 83.7, 125.0, 125.7, 126.4, 126.5, 126.6, 126.9, 127.0, 127.37, 127.41, 127.6, 127.8, 127.9, 128.0, 128.1, 129.2, 130.2, 131.5, 133.0, 133.5, 133.8, 142.2, 144.4, 146.2, 147.2; IR (KBr) 1623, 2925, 3016, 3060, 3379, 3491 cm⁻¹; MS (FAB) m/z 451 (M)⁺, 452 (M+H)⁺; HRMS (FAB) m/z calcd for C₃₃H₂₅NO (M)⁺ 451.1936, found 451.1929.

### Example 3

### Synthesis of 3-(pyridin-4-yl)dibenzo[a,k]phenanthridin-4(3H)-one (18)

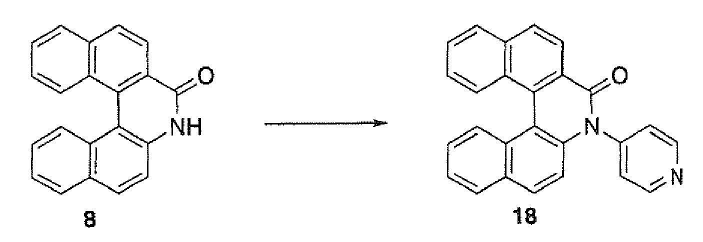

Diglyme (1.0 mL) was added to dibenzo[a,k]phenanthridin-4(3H)-one (8) (20 mg, 0.07 mmol), 4-bromopyridine HCl salt (41 mg, 0.21 mmol)), and Cs₂CO₃ (91 mg, 0.28 mmol) in an Ar atmosphere, and the mixture was stirred at room temperature for 5 minutes. Subsequently, CuI (20 mg, 0.11 mmol), N,N'-dimethylethylenediamine (15 µL, 0.14 mmol), and DMF (3.0 mL) were added to the mixture, and the resulting mixture was stirred at 120°C for 27 hours. After adding a saturated ammonium chloride aqueous solution to the reaction liquid, extraction using ethyl acetate was performed. The resulting organic layer was washed with a saturated NaHCO₃ aqueous solution and a saturated sodium chloride solution, dried over Na₂SO₄, and the solvent was then distilled off under reduced pressure. The residue was purified using silica gel column chromatography (hexane:ethyl acetate = 1:1), obtaining 3-(pyridin-4-yl)dibenzo[a,k]phenanthridin-4(3H)-one (18). (13 mg, 50%). ¹H NMR (400 MHz, CDCl₃) δ 6.93 (d, J= 8.7 Hz, 1H), 7.30-7.53 (m, 5H), 7.62-7.70 (m, 2H), 7.84 (d, J = 8.7 Hz, 1H), 7.89 (d, J = 8.2 Hz, 1H), 7.97 (d, J = 8.7 Hz, 1H), 8.03 (d, J = 8.3 Hz, 1H), 8.08 (d, J = 8.7 Hz, 1H), 8.14 (d, J = 8.7 Hz, 1H), 8.50 (d, J = 8.7 Hz, 1H), 8.94 (d, J = 5.5 Hz, 1H); MS (FAB) m/z 373 (M+H)⁺; HRMS (FAB) m/z calcd for C₂₆H₁₇NO₂ (M+H)⁺ 373.1341, found 373.1347.

### Synthesis of 2'-(pyridin-4-ylamino)-1,1'-binaphthyl-2-carboxylic acid (19)

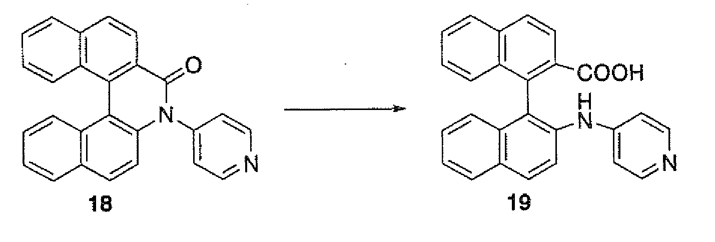

NaOH (110 mg, 2.74 mmol) and DMSO (1.5 mL) were added to 3-(pyridin-4-yl)dibenzo[a,k]phenanthridin-4(3H)-one (18) (102 mg, 0.27 mmol) at room temperature, and the mixture was stirred at 70°C for 1 hour. NaOH (60 mg, 1.50 mmol) was further added thereto, and the resulting mixture was stirred at 70°C for 30 minutes. Water (7.0 mL) was added to the reaction liquid, and neutralized with 1N aq. HCl, a saturated sodium chloride solution (10 mL) was added thereto, and extraction was performed using chloroform. The resulting organic layer was washed with a saturated sodium chloride solution and dried over Na₂SO₄. Thereafter, the solvent was distilled off under reduced pressure. The residue was washed with diethyl ether, obtaining 2'-(pyridin-4-ylamino)-1,1'-binaphthyl-2-carboxylic acid (19) (40 mg, 38%).
¹H NMR (400 MHz, CD₃OD) δ 6.84 (d, J = 6.4 Hz, 1H), 6.99 (d, J = 8.7 Hz, 1H), 7.06 (d, J = 8.7 Hz, 1H), 7.10-7.24 (m, 2H), 7.37 (d, J = 7.8 Hz, 1H), 7.41 (d, J = 6.9 Hz, 1H), 7.56 (d, J = 8.7 Hz, 1H), 7.72-7.93 (m, 6H), 7.98 (d, J = 8.7 Hz, 1H), 8.02 (d, J = 8.7 Hz, 1H); MS (FAB) m/z 391 (M+H)⁺. Acylation reaction using 2'-(pyridin-4-ylamino)-1,1'-binaphthyl-2-carboxylic acid (19) as a catalyst

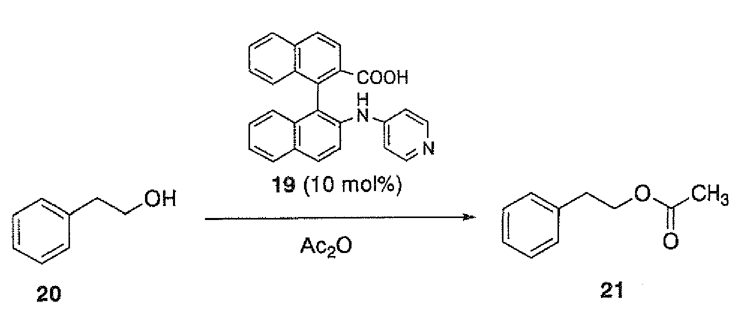

2'-(Pyridin-4-ylamino)-1,1'-binaphthyl-2-carboxylic acid (19) (10 mg, 10 mol%), AcONa (2.0 g, 23 mmol), and 2-phenylethanol (20) (30 µL, 0.25 mmol) were mixed and stirred at room temperature for 17 hours. After filtering the reaction liquid, the solvent was distilled off under reduced pressure. The residue was purified using preparative thin layer chromatography (hexane:ethyl acetate = 2:1), obtaining phenethyl acetate (21) (23 mg, 56%).
¹H NMR (400 MHz, CDCl₃) 5 2.03 (s, 3H), 2.93 (t, J= 7.3 Hz, 2H), 4.28 (t, J = 7.3 Hz, 2H), 7.18-7.26 (m, 3H), 7.26-7.34 (m, 2H).

### Example 4

### 2'-(tert-Butoxycarbonyl(methy)amino)-1,1'-binaphthyl-2-carboxylic acid (22)

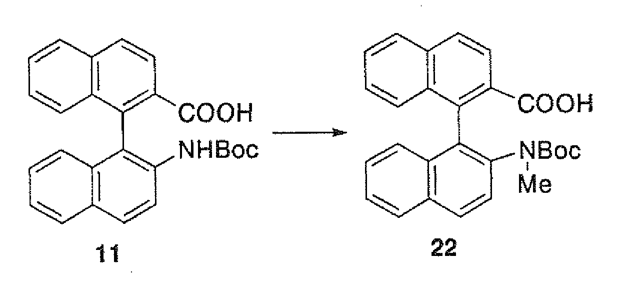

THF (6.0 mL) was added to 2'-(test-butoxycarbonylamino)-1,1'-binaphthyl-2-carboxylic acid (11) (297 mg, 0.72 mmol) and NaH (60%) (72 mg, 1.8 mmol) at 0°C in a nitrogen atmosphere, and the mixture was stirred at room temperature for 12 hours. After adding a saturated ammonium chloride aqueous solution, the reaction liquid was subjected to extraction using ethyl acetate. The water layer was then extracted using ethyl acetate, and the collected organic layers were washed with a saturated sodium chloride solution. The organic layers were dried over Na₂SO₄, and the solvent was distilled off under reduced pressure. The residue was recrystallized using hexane-ethyl acetate, obtaining 2'-(tert-butoxycarbonyl(methyl)amino)-1,1'-binaphthyl-2-carboxylic acid (22) (158 mg). Furthermore, the filtrate was purified using silica gel column chromatography (chloroform→chloroform:methanol = 10:1), obtaining 2'-(tert-butoxycarbonyl(methyl)amino)-1,1'-binaphthyl-2-carboxylic acid (22) (64 mg). The total amount of 2'-(tert-butoxycarbonyl(methyl)amino)-1,1'-binaphthyl-2-carboxylic acid (22) obtained was 222 mg (72%).
¹H NMR (400 MHz, CDCl₃) δ 1.08 (s, 9H), 2.98 (s, 3H), 7.22-7.41 (m, 4H), 7.45 (d, J = 8.7 Hz, 1H), 7.48-7.60 (m, 2H), 7.85 (d, J = 8.2 Hz, 1H), 7.92 (d, J = 8.2 Hz, 1H), 7.96 (d, J = 8.2 Hz, 1H), 7.99 (d, J = 8.2 Hz, 2H), 8.06 (d, J = 8.7 Hz, 1H); MS (FAB) m/z 427 (M)⁺, 428 (M+H)⁺, 450 (M+Na)⁺; HRMS (FAB) m/z calcd for C₂₇H₂₅NO₄ (M)⁺ 427.1784, found 427.1784.

### Synthesis of compounds 22 to 25

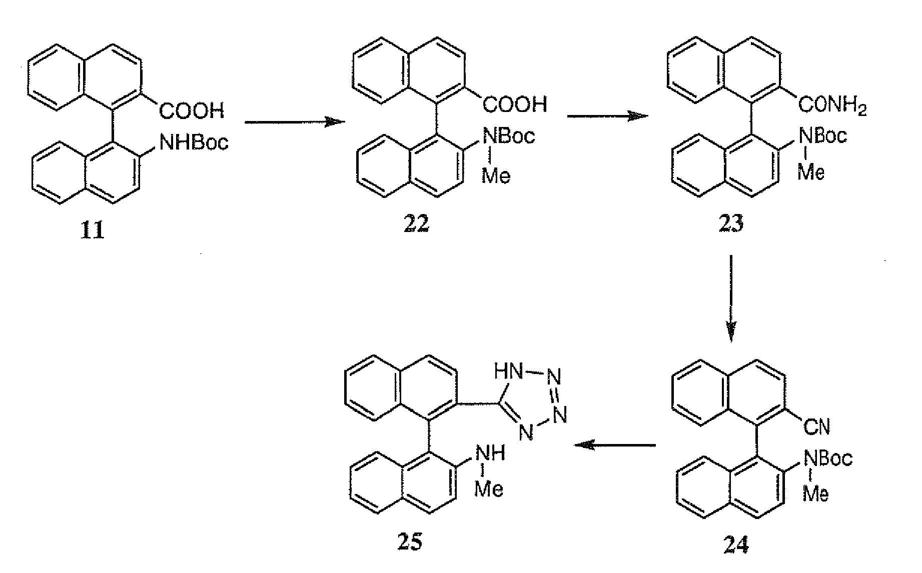

Example 4 shows the synthetic process of compound 22. When compound 22 is allowed to react according to a conventional method, in the presence of a coupling agent (for example, DCC and carbonyldiimidazole) and ammonia at about room temperature for about 1 to 24 hours, in the presence of a solvent (for example, DMF, THF, and dichloromethane), amide compound 23 can be obtained. 1-Hydroxybenzotriazole (HOBt) may be added thereto. Subsequently, the amide compound 23 can be converted to a CN-containing compound (compound 24) by treating it with diphosphorus pentaoxide and like dehydrating agents. The nitrile (CN) of compound 24 can be converted to tetrazole by a [3+2] cycloaddition reaction with an azide. The tetrazole compound 24 is useful as a reaction catalyst for asymmetric synthesis.

### tert-Butyl 2'-carbamoyl-1,1-binaphthyl-2-yl(methyl)carbamate (23)

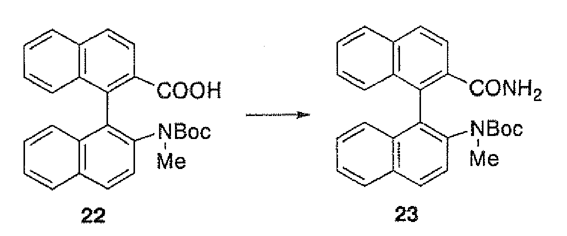

In an argon atmosphere, 2'-(tert-butoxycarbonyl(methyl)amino)-1,1'-binaphthyl-2-carboxylic acid (22) (100 mg, 0.23 mmol), EDCI·HCl (90 mg, 0.47 mmol), and HOBt (96 mg, 0.70 mmol) were stirred in THF (2.0 mL) at room temperature for 30 minutes. Aqueous ammonia (4.0 mL) was added to the reaction liquid, and the mixture was stirred at room temperature for 2 hours. After adding a saturated ammonium chloride aqueous solution to the reaction liquid, extraction using ethyl acetate was conducted. Thereafter, the water layer was further extracted using ethyl acetate, and the collected organic layers were washed with a saturated sodium chloride solution. The resulting organic layers were dried over Na₂SO₄, and then the solvent was distilled off under reduced pressure. The residue was recrystallized using hexane-ethyl acetate, obtaining 2'-(tert-butoxycarbonyl(methyl)amino)-1,1'-binaphthyl-2-carboxylic acid (22) (158 mg). Subsequently, the filtrate was purified using silica gel column chromatography (chloroform-chloroform:methanol = 10:1), obtaining tert-butyl 2'-carbamoyl-1,1'-binaphthyl-2-yl(methyl)carbamate (23) (90 mg, 91%). ¹H NMR (400 MHz, CDCl₃) δ 1.12 (s, 9H), 2.86 (s, 3H), 5.08 (br s, 1H), 7.10-7.60 (m, 6H), 7.80-8.10 (m, 6H); MS (FAB) m/z 427 (M+H)⁺, 449 (M+Na)⁺.

### tert-Butyl 2'-cyano-1,1'-binaphthyl-2-yl(methyl)carbamate (24)

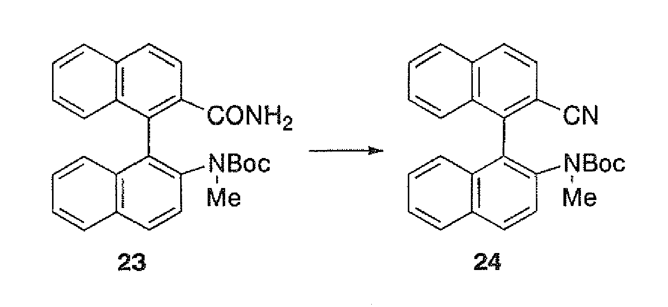

In an argon atmosphere, pyridine (60 µL, 0.77 mmol) was added to a CH₂Cl₂ solution (2.0 mL) of tert-butyl 2'-carbamoyl-1,1'-binaphthyl-2-yl(methyl)arbamate (23) (50 mg, 0.12 mmol) and TsCl (90 mg, 0.47 mmol), and the mixture was stirred at room temperature for 17 hours. Aqueous ammonia (4.0 mL) was added to the reaction liquid, and the resulting mixture was stirred at room temperature for 2 hours. After adding a saturated NaHCO₃ aqueous solution to the reaction liquid, extraction using ethyl acetate was conducted. The water layer was further extracted using ethyl acetate, and the collected organic layers were washed with a saturated sodium chloride solution. The resulting organic layers were dried over Na₂SO₄, and the solvent was distilled off under reduced pressure. The residue was purified using silica gel column chromatography (hexane:ethyl acetate = 40:1→20:15:1), obtaining tert-butyl 2'-cyano-1,1'-binaphthyl-2-yl(methyl)carbamate (24) (47 mg, 98%).
¹H NMR (400 MHz, CDCl₃) δ 1.26 (br s, 9H), 2.94 (br s, 3H), 6.90-7.15 (m, 1H), 7. 78 (d, J = 8.7 Hz, 1H), 7.96 (d, J = 8.2 Hz, 1H), 8.03 (d, J = 8.7 Hz, 1H); MS (FAB) m/z 409 (M+H)⁺, 431 (M+Na)⁺.

### N-Methyl-2'-(1H-tetrazol-5-yl)-1,1-binaphthyl-2-amine (25)

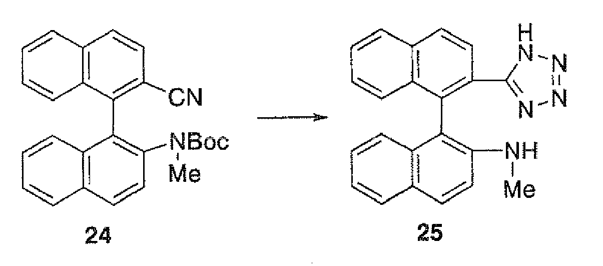

In an argon atmosphere, n-Bu₃SnCl (36 µL, 0.13 mmol) was added to a xylene solution (1.0 mL) of tert-butyl 2'-cyano-1,1'-binaphthyl-2-yl(methyl)carbamate (24) (24 mg, 0.059 mmol) and NaN₃ (15 mg, 0.12 mmol), and the mixture was stirred at 140°C for 45 hours. Water was added to the reaction liquid, and then extraction using ethyl acetate (100 mL) was conducted. 4 N HCl ethyl acetate (100 mL) was added to the ethyl acetate layer, and the resulting mixture was stirred for 30 minutes. Subsequently, the pH was adjusted to 10 using NaHCO₃ and a 1 N NaOH aqueous solution under ice cooling, followed by extraction using water. The pH of the water layer was adjusted to 2 using a 2 N HCl aqueous solution, followed by extraction using ethyl acetate. The resulting organic layer was washed with a saturated sodium chloride solution, dried over Na₂SO₄, and then the solvent was distilled off under reduced pressure. Subsequently, the residue was purified using silica gel column chromatography (chloroform), obtaining N-methyl-2'-(1H-tetrazol-5-yl)-1,1'-binaphthyl-2-amine (25) (10 mg, 48 %).
¹H NMR (400 MHz, CDCl₃) δ 2.80 (s, 3H), 6.72 (d, J = 8.2 Hz, 1H), 7.10-7.45 (m, 5H), 7.55-7.65 (m, 1H), 7.85 (d, J = 7.8 Hz, 1H), 8.00-8.10 (m, 2H), 8.18 (d, J = 8.7 Hz, 1H), 8.58 (d, J = 8.7 Hz, ¹H) ; ¹³C NMR (100 MHz, CDCl₃) δ 30.8, 112.4, 125.7, 113.3, 122.1, 122.9, 123.1, 126.48, 126.51, 127.4, 127.8, 128.1, 128.4, 128.50, 128.55, 129.8, 131.5, 132.6, 132.9, 133.6, 135.3, 144.4, 153.8; MS (FAB) m/z 352 (M+H)⁺, 374 (M+Na)⁺; HRMS (FAB) m/z calcd for C₂₂H₁₈N₅ (M+H)⁺ 352.1562, found 352.1587.

### Industrial Applicability

In the development of fine chemicals such as medicines and organic materials, asymmetric synthesis that makes it possible to accurately obtain different forms of optically active substances serves an extremely important role. Axially chiral compounds have been used as effective asymmetric sources for achieving such an important asymmetric synthesis. In addition, amino acids and amino alcohols also comprise a large category as important asymmetric sources.

The axially chiral amino acids and amino alcohols of the present invention have a high market value as they hybridize the features of axially chiral compounds and the features of amino acids and amino alcohols.

The compound of the present invention is useful for medicinal intermediates, optical active materials and the like as a chiral building block.

## Claims

1. A helicene derivative or salt thereof represented by Formula (I) or (I'): 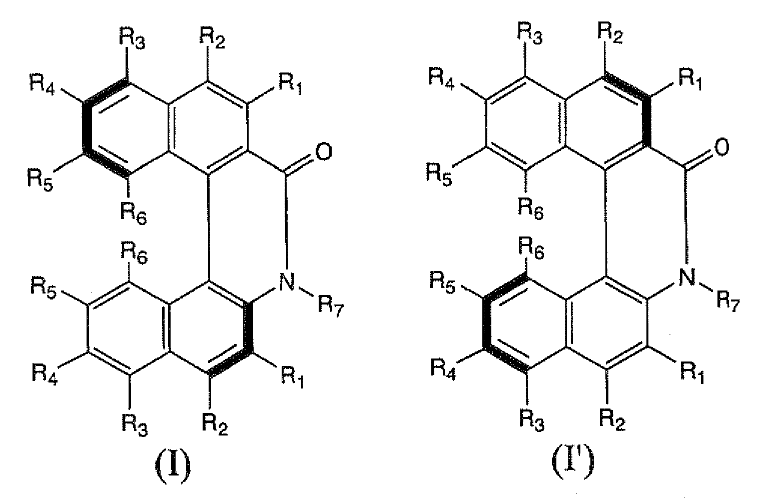 wherein
R₁, R₂, R₃, R₄, R₅, and R₆ may be the same or different, and represent hydrogen, alkyl, cycloalkyl, aryl, heteroaryl, alkenyl, alkynyl, cyano, alkoxy, hydroxy, acyloxy, CONH₂, monoalkylaminocarbonyl, dialkylaminocarbonyl, nitro, amino, monoalkylamino, dialkylamino, alkanoylamino, azido, carboxy (COOH), alkoxycarbonyl, or acyl; two adjacent groups among R₁, R₂, R₃, R₄, R₅, and R₆ may be joined together with the carbon atoms to which they are attached to form a 5- or 6-membered saturated or unsaturated ring structure or structures, and these ring structures may be substituted; and R₇ represents hydrogen, alkyl, cycloalkyl, aryl, heteroaryl, acyl, or alkoxycarbonyl.

2. An axially chiral amino acid, amine or amino alcohol derivative, or salt thereof, represented by Formula (II) or (II'): 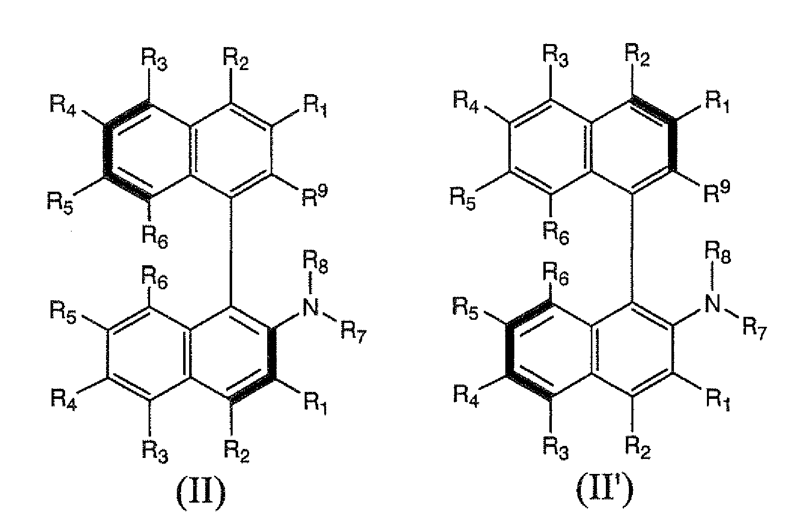 wherein
R₁, R₂, R₃, R₄, R₅, and R₆ may be the same or different, and represent hydrogen, alkyl, cycloalkyl, aryl, heteroaryl, alkenyl, alkynyl, cyano, alkoxy, hydroxy, acyloxy, CONH₂, monoalkylaminocarbonyl, dialkylaminocarbonyl, nitro, amino, monoalkylamino, dialkylamino, alkanoylamino, azido, carboxy (COOH), alkoxycarbonyl, or acyl; two adjacent groups among R₁, R₂, R₃, R₄, R₅, and R₆ may be joined together with the carbon atoms to which they are attached to form a 5- or 6-membered saturated or unsaturated ring structure or structures, and these ring structures may be substituted; R₇ and R₈ each independently represent hydrogen, alkyl, aryl, heteroaryl, acyl, or alkoxycarbonyl; and R₉ represents COOZ, CONZ₂ or CZ₂OH (each Z is the same or different, and represents hydrogen, alkyl, cycloalkyl, aryl, heteroaryl, or aralkyl), CN, or tetrazole.

3. A perylene derivative or salt thereof represented by Formula (III): 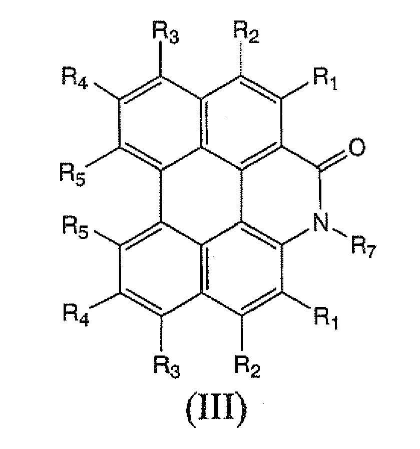 wherein
R₁, R₂, R₃, R₄, and R₅ may be the same or different, and represent hydrogen, alkyl, cycloalkyl, aryl, heteroaryl, alkenyl, alkynyl, cyano, alkoxy, hydroxy, acyloxy, CONH₂, monoalkylaminocarbonyl, dialkylaminocarbonyl, nitro, amino, monoalkylamino, dialkylamino, alkanoylamino, azido, carboxy (COOH), alkoxycarbonyl, or acyl; two adjacent groups among R₁, R₂, R₃, R₄, and R₅ may be joined together with the carbon atoms to which they are attached to form a 5- or 6-membered saturated or unsaturated ring structure or structures, and these ring structures may be substituted; and R₇ represents hydrogen, alkyl, cycloalkyl, aryl, heteroaryl, acyl, or alkoxycarbonyl.

4. A method for producing a helicene derivative represented by Formula (I) or (I'): 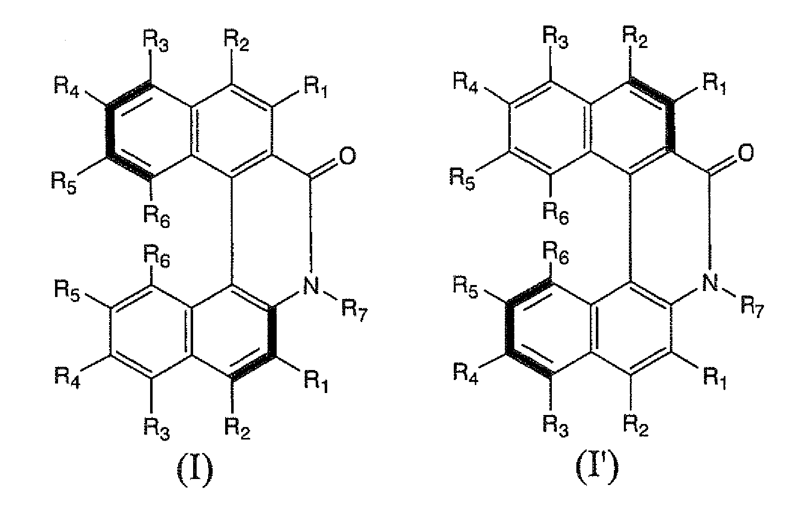 wherein R₁ to R₇ are as defined below,
the method comprising reacting, with a Pd catalyst, a compound represented by Formula (IV): 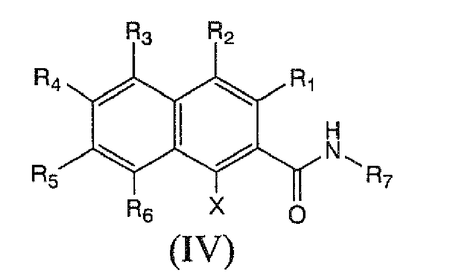 wherein
R₁, R₃, R₄, R₅, and R₆ may be the same or different, and represent hydrogen, alkyl, cycloalkyl, aryl, heteroaryl, alkenyl, alkynyl, cyano, alkoxy, hydroxy, acyloxy, CONH₂, monoalkylaminocarbonyl, dialkylaminocarbonyl, nitro, amino, monoalkylamino, dialkylamino, alkanoylamino, azido, carboxy (COOH), alkoxycarbonyl, or acyl; two adjacent groups among R₁, R₂, R₃, R₄, R₅, and R₆ may be joined together with the carbon atoms to which they are attached to form a 5- or 6-membered saturated or unsaturated ring structure or structures, and these ring structures may be substituted; R₇ represents hydrogen, alkyl, cycloalkyl, aryl, heteroaryl, acyl, or alkoxycarbonyl; and X represents iodine, bromine, chlorine, or triflate.

5. A method for producing an axially chiral amino acid, amine, or amino alcohol derivative represented by Formula (II) or (II'): 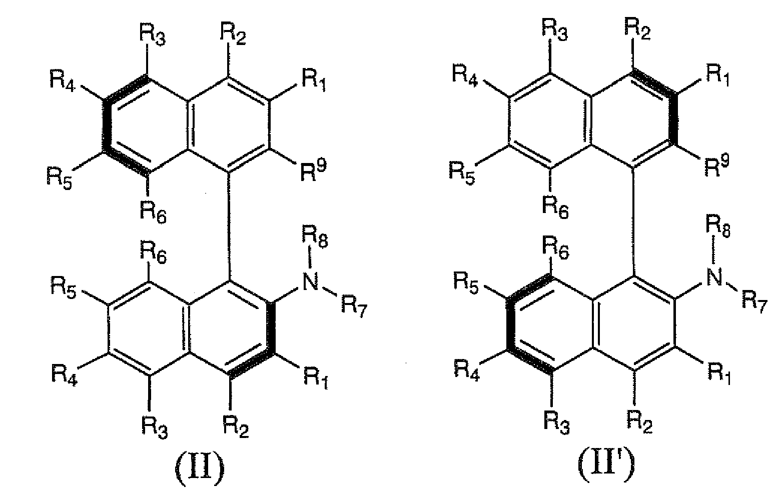 wherein
R_{1,} R₂, R₃, R₄, R₅, and R₆ may be the same or different, and represent hydrogen, alkyl, cycloalkyl, aryl, heteroaryl, alkenyl, alkynyl, cyano, alkoxy, hydroxy, acyloxy, CONH₂, monoalkylaminocarbonyl, dialkylaminocarbonyl, nitro, amino, monoalkylamino, dialkylamino, alkanoylamino, azido, carboxy (COOH), alkoxycarbonyl, or acyl; two adjacent groups among R₁, R₂, R₃, R₄, R₅, and R₆ may be joined together with the carbon atoms to which they are attached to form a 5- or 6-membered saturated or unsaturated ring structure or structures, and these ring structures may be substituted; R₇ and R₈ each independently represent hydrogen, alkyl, aryl, heteroaryl, acyl, or alkoxycarbonyl; and R₉ represents COOZ, CONZ₂ or CZ₂OH (each Z is the same or different, and represents hydrogen, alkyl, cycloalkyl, aryl, heteroaryl, or aralkyl), CN, or tetrazole,
the method comprising opening a lactam ring in the compound represented by Formula (I) or (I') in Claim 1, using a metal hydroxide; converting, if necessary, a resulting carboxy (COOH) to COOZ, CONZ₂ or CZ₂OH (each Z is the same or different, and represents hydrogen, alkyl, cycloalkyl, aryl, heteroaryl, or aralkyl), CN, or tetrazole; and converting, if necessary, an amino group (NH₂) to NR₇R₈, wherein R₇ and R₈ each independently represent hydrogen, alkyl, aryl, heteroaryl, acyl, or alkoxycarbonyl.

6. A method for converting, using a sulfonic acid, a compound represented by Formula (Ia) or (Ia'): 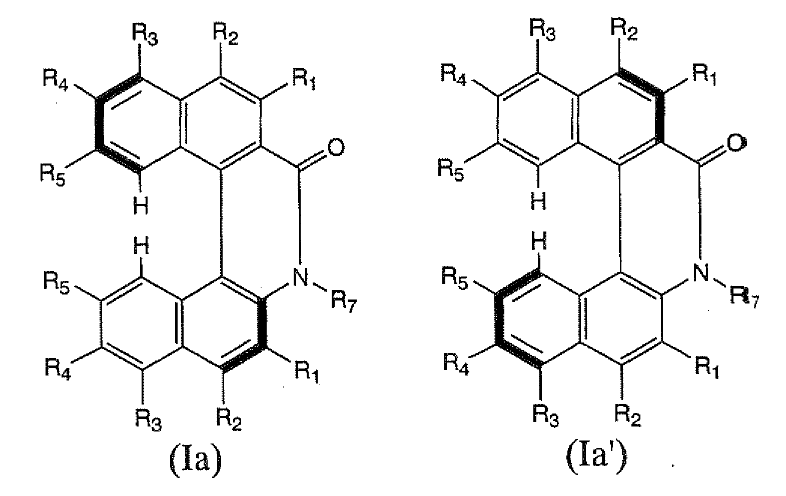 wherein
R₁, R₂, R₃, R_{4,} and R₅ may be the same or different, and represent hydrogen, alkyl, cycloalkyl, aryl, heteroaryl, alkenyl, alkynyl, cyano, alkoxy, hydroxy, acyloxy, CONH₂, monoalkylaminocarbonyl, dialkylaminocarbonyl, nitro, amino, monoalkylamino, dialkylamino, alkanoylamino, azido, carboxy (COOH), alkoxycarbonyl, or acyl; two adjacent groups among R₁, R₂, R₃, R₄, and R₅ may be joined together with the carbon atoms to which they are attached to form a 5- or 6-membered saturated or unsaturated ring structure or structures, and these ring structures may be substituted; and R₇ represents hydrogen, alkyl, cycloalkyl, aryl, heteroaryl, acyl, or alkoxycarbonyl,
to a perylene derivative represented by Formula (III): 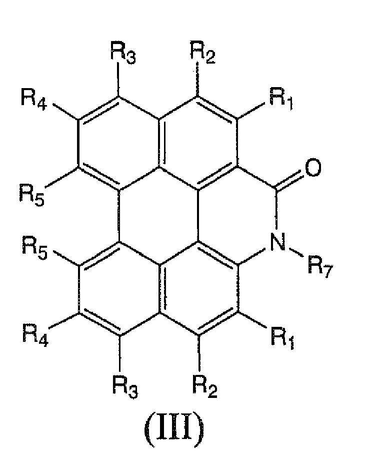 wherein R₁ to R₅ and R₇ are as defined above.
